# EUROPEAN PATENT APPLICATION

(11) **EP 4 180 457 A1**
(43) Date of publication of application: **17.05.2023**
(21) Application number: 21842171.7
(22) Date of filing: 13.07.2021
(51) Int. Cl.: C07K 16/28, A61K 39/395, A61P 35/00, A61P 35/02

(54) **ANTI-CLDN-18.2 ANTIBODY AND USE THEREOF**

(30) Priority: 13.07.2020 CN 202010671413
(71) Applicant: Shanghai Junshi Biosciences Co., Ltd., Pilot Free Trade Zone Shanghai 201210 (CN); Suzhou Junmeng Biosciences Co., Ltd., Suzhou, Jiangsu 215002 (CN)
(72) Inventor: ZHOU, Yuehua, Suzhou, Jiangsu 215002 (CN); ZHANG, Jing, Suzhou, Jiangsu 215002 (CN); LIU, Hui, Suzhou, Jiangsu 215002 (CN); LIU, Hongchuan, Suzhou, Jiangsu 215002 (CN); WU, Hai, Suzhou, Jiangsu 215002 (CN); YAO, Jian, Suzhou, Jiangsu 215002 (CN); HUANG, Lanqing, Suzhou, Jiangsu 215002 (CN)
(74) Representative: Cabinet Becker et Associés
(86) International application number: PCT/CN2021/106125
(87) International publication number: WO 2022/012559

(57) **Abstract**

Provided are a CLDN-18.2 specific binding antibody or an antigen binding fragment thereof, and a composition comprising same. Also provided are a nucleic acid molecule coding the antibody or the antigen binding fragment thereof, an expression vector and a host cell for expressing the antibody or the antigen binding fragment thereof, and a therapeutic or diagnostic method and use for the antibody or the antigen binding fragment thereof.

## Description

### Technical Field

The present invention provides a CLDN-18.2 specific binding antibody or an antigen binding fragment thereof, and a composition comprising same. Also provided are a nucleic acid molecule coding the antibody or the antigen binding fragment thereof of the present invention, an expression vector and a host cell for expressing the antibody or the antigen binding fragment thereof of the present invention, and a therapeutic or diagnostic method and use for the antibody or the antigen binding fragment thereof of the present invention.

### Background Art

Gastric cancer is one of the most prevalent cancers worldwide. According to statistics from the World Health Organization's cancer control project, up to 7 million patients die of cancer worldwide each year, of which 700,000 patients die from gastric cancer. Compared with conventional gastric cancer treatment regimens, antibody-based treatment regimens have far-reaching application prospects due to their high specificity and low side effects.

Claudin, also known as CLDN, is a family of cell surface proteins that establish paracellular barriers and control the flow of molecules between cells. At least 26 Claudin species have been discovered so far. Members of the Claudin protein family are important structural components of tight junctions, which play an important role in maintaining epithelial cell polarity, controlling paracellular diffusion, and regulating cell growth and differentiation. Claudin molecules cross the cell membrane four times, with both the N-terminus and the C-terminus in the cytoplasm. Different Claudin members are expressed in different tissues, and alterations in their function are associated with cancer formation. Changes in the expression levels of Claudin 1, Claudin 18, and Claudin 10 are associated with intestinal cancer, gastric cancer, and hepatocellular carcinoma, respectively.

Claudin 18 (CLDN18) has two alternative splicing variants, CLDN-18.1 and CLDN-18.2. Claudin 18.1 (CLDN-18.1) is selectively expressed in normal lung and gastric epithelium. Claudin 18.2 (CLDN-18.2) shows trace expression in normal gastric epithelial short-lived cells, but in tumour cells, Claudin 18.2 shows strong expression in various cancer types, for example, Claudin 18.2 is highly expressed in 75% of patients with gastric cancer, 50% of patients with pancreatic cancer and 30% of patients with esophageal cancer, and also in lung cancer and so on.

Although Claudiximab (IMAB362) developed by Ganymed is currently available in phase II clinical trials for advanced gastroesophageal cancer (WO 2007059997), there is still a need for a novel anti-CLDN18.2 antibody with improved affinity and specificity compared with known antibodies.

### Summary of the Invention

The present invention provides an anti-CLDN-18.2 antibody or an antigen binding fragment thereof, which has the advantages of high affinity and high specificity for human CLDN-18.2. The anti-CLDN-18.2 antibody or the antigen binding fragment thereof provided by the present invention can be used as an independent therapy or in combination with other therapies and/or other anti-cancer agents for the treatment of, for example, a cancer.

In one aspect, the present invention provides an anti-CLDN-18.2 antibody or an antigen binding fragment thereof comprising a heavy chain variable region and a light chain variable region, wherein,
the heavy chain variable region comprises HCDR1, HCDR2 and HCDR3, wherein
   the sequence of the HCDR1 is selected from amino acid sequences as shown in SEQ ID NOs: 1, 10, 16, 22, 28, 34 and 37;
   the sequence of the HCDR2 is selected from amino acid sequences as shown in SEQ ID NOs: 62, 63, 17, 23, 29, 35, 38, 72 and 74; in the HCDR2 as shown in SEQ ID NO: 62, X₁ is C or S, and X₂ is T or S, and in the HCDR2 as shown in SEQ ID NO:63, X₃ is D or G, and X₄ is K or T; and
   the sequence of the HCDR3 is selected from amino acid sequences as shown in SEQ ID NOs: 3, 12, 18, 24, 30, 36, 39, 73 and 75; and
the light chain variable region comprises LCDR1, LCDR2 and LCDR3, wherein
   the sequence of the LCDR1 is selected from amino acid sequences as shown in SEQ ID NOs: 4, 7, 13, 19, 25 and 31;
   the sequence of the LCDR2 is selected from amino acid sequences as shown in SEQ ID NOs: 5, 8, 14, 20, 26 and 32; and
   the sequence of the LCDR3 is selected from amino acid sequences as shown in SEQ ID NOs: 6, 9, 15, 21, 27 and 33.

In some embodiments, the heavy chain variable region of the present invention comprises:
(I) HCDR1, HCDR2 and HCDR3 with amino acid sequences as shown in SEQ ID NO: 1, SEQ ID NO: 2 and SEQ ID NO: 3, respectively; or
(II) HCDR1, HCDR2 and HCDR3 with amino acid sequences as shown in SEQ ID NO: 10, SEQ ID NO: 11 and SEQ ID NO: 12, respectively; or
(III) HCDR1, HCDR2 and HCDR3 with amino acid sequences as shown in SEQ ID NO: 16, SEQ ID NO: 17 and SEQ ID NO: 18, respectively; or
(IV) HCDR1, HCDR2 and HCDR3 with amino acid sequences as shown in SEQ ID NO: 22, SEQ ID NO: 23 and SEQ ID NO: 24, respectively; or
(V) HCDR1, HCDR2 and HCDR3 with amino acid sequences as shown in SEQ ID NO: 28, SEQ ID NO: 29 and SEQ ID NO: 30, respectively; or
(VI) HCDR1, HCDR2 and HCDR3 with amino acid sequences as shown in SEQ ID NO: 34, SEQ ID NO: 35 and SEQ ID NO: 36, respectively; or
(VII) HCDR1, HCDR2 and HCDR3 with amino acid sequences as shown in SEQ ID NO: 37, SEQ ID NO: 38 and SEQ ID NO: 39, respectively; or
(VIII) HCDR1, HCDR2 and HCDR3 with amino acid sequences as shown in SEQ ID NO: 10, SEQ ID NO: 40 and SEQ ID NO: 12, respectively; or
(IX) HCDR1, HCDR2 and HCDR3 with amino acid sequences as shown in SEQ ID NO: 1, SEQ ID NO: 41 and SEQ ID NO: 3, respectively; or
(X) HCDR1, HCDR2 and HCDR3 with amino acid sequences as shown in SEQ ID NO: 1, SEQ ID NO: 72 and SEQ ID NO: 73, respectively; or
(XI) HCDR1, HCDR2 and HCDR3 with amino acid sequences as shown in SEQ ID NO: 1, SEQ ID NO: 72 and SEQ ID NO: 3, respectively; or
(XII) HCDR1, HCDR2 and HCDR3 with amino acid sequences as shown in SEQ ID NO: 28, SEQ ID NO: 74 and SEQ ID NO: 75, respectively; or
(XIII) HCDR1, HCDR2 and HCDR3 with amino acid sequences as shown in SEQ ID NO: 28, SEQ ID NO: 74 and SEQ ID NO: 30, respectively; and
the light chain variable region comprises:
(I) LCDR1, LCDR2 and LCDR3 with amino acid sequences as shown in SEQ ID NO: 4, SEQ ID NO: 5 and SEQ ID NO: 6, respectively; or
(II) LCDR1, LCDR2 and LCDR3 with amino acid sequences as shown in SEQ ID NO: 7, SEQ ID NO: 8 and SEQ ID NO: 9, respectively; or
(III) LCDR1, LCDR2 and LCDR3 with amino acid sequences as shown in SEQ ID NO: 13, SEQ ID NO: 14 and SEQ ID NO: 15, respectively; or
(IV) LCDR1, LCDR2 and LCDR3 with amino acid sequences as shown in SEQ ID NO: 19, SEQ ID NO: 20 and SEQ ID NO: 21, respectively; or
(V) LCDR1, LCDR2 and LCDR3 with amino acid sequences as shown in SEQ ID NO: 25, SEQ ID NO: 26 and SEQ ID NO: 27, respectively; or
(VI) LCDR1, LCDR2 and LCDR3 with amino acid sequences as shown in SEQ ID NO: 31, SEQ ID NO: 32 and SEQ ID NO: 33, respectively.

In some embodiments, the antibody or the antigen binding fragment thereof of the present invention comprises:
(I) a heavy chain variable region comprising HCDR1, HCDR2 and HCDR3 with amino acid sequences as shown in SEQ ID NO: 1, SEQ ID NO: 2 and SEQ ID NO: 3, respectively; and a light chain variable region comprising LCDR1, LCDR2 and LCDR3 with amino acid sequences as shown in SEQ ID NO: 4, SEQ ID NO: 5 and SEQ ID NO: 6, respectively; or
(II) a heavy chain variable region comprising HCDR1, HCDR2 and HCDR3 with amino acid sequences as shown in SEQ ID NO: 1, SEQ ID NO: 2 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(III) a heavy chain variable region comprising HCDR1, HCDR2 and HCDR3 with amino acid sequences as shown in SEQ ID NO: 10, SEQ ID NO: 11 and SEQ ID NO: 12, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(IV) a heavy chain variable region comprising HCDR1, HCDR2 and HCDR3 with amino acid sequences as shown in SEQ ID NO: 16, SEQ ID NO: 17 and SEQ ID NO: 18, respectively; and a light chain variable region comprising LCDR1, LCDR2 and LCDR3 with amino acid sequences as shown in SEQ ID NO: 19, SEQ ID NO: 20 and SEQ ID NO: 21, respectively; or
(V) a heavy chain variable region comprising HCDR1, HCDR2 and HCDR3 with amino acid sequences as shown in SEQ ID NO: 22, SEQ ID NO: 23 and SEQ ID NO: 24, respectively; and a light chain variable region comprising LCDR1, LCDR2 and LCDR3 with amino acid sequences as shown in SEQ ID NO: 25, SEQ ID NO: 26 and SEQ ID NO: 27, respectively; or
(VI) a heavy chain variable region comprising HCDR1, HCDR2 and HCDR3 with amino acid sequences as shown in SEQ ID NO: 28, SEQ ID NO: 29 and SEQ ID NO: 30, respectively; and a light chain variable region comprising LCDR1, LCDR2 and LCDR3 with amino acid sequences as shown in SEQ ID NO: 31, SEQ ID NO: 32 and SEQ ID NO: 33, respectively; or
(VII) a heavy chain variable region comprising HCDR1, HCDR2 and HCDR3 with amino acid sequences as shown in SEQ ID NO: 34, SEQ ID NO: 35 and SEQ ID NO: 36, respectively; and a light chain variable region comprising LCDR1, LCDR2 and LCDR3 with amino acid sequences as shown in SEQ ID NO: 25, SEQ ID NO: 26 and SEQ ID NO: 27, respectively; or
(VIII) a heavy chain variable region comprising HCDR1, HCDR2 and HCDR3 with amino acid sequences as shown in SEQ ID NO: 37, SEQ ID NO: 38 and SEQ ID NO: 39, respectively; and a light chain variable region comprising LCDR1, LCDR2 and LCDR3 with amino acid sequences as shown in SEQ ID NO: 31, SEQ ID NO: 32 and SEQ ID NO: 33, respectively; or
(IX) a heavy chain variable region comprising HCDR1, HCDR2 and HCDR3 with amino acid sequences as shown in SEQ ID NO: 10, SEQ ID NO: 11 and SEQ ID NO: 12, respectively; and a light chain variable region comprising LCDR1, LCDR2 and LCDR3 with amino acid sequences as shown in SEQ ID NO: 19, SEQ ID NO: 20 and SEQ ID NO: 21, respectively; or
(X) a heavy chain variable region comprising HCDR1, HCDR2 and HCDR3 with amino acid sequences as shown in SEQ ID NO: 28, SEQ ID NO: 29 and SEQ ID NO: 30, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XI) a heavy chain variable region comprising HCDR1, HCDR2 and HCDR3 with amino acid sequences as shown in SEQ ID NO: 22, SEQ ID NO: 23 and SEQ ID NO: 24, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XII) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XIII) a heavy chain variable region comprising HCDR1, HCDR2 and HCDR3 with amino acid sequences as shown in SEQ ID NO: 1, SEQ ID NO: 41 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(XIV) a heavy chain variable region comprising HCDR1, HCDR2 and HCDR3 with amino acid sequences as shown in SEQ ID NO: 1, SEQ ID NO: 72 and SEQ ID NO: 73, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(XV) a heavy chain variable region comprising HCDR1, HCDR2 and HCDR3 with amino acid sequences as shown in SEQ ID NO: 1, SEQ ID NO: 72 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(XVI) a heavy chain variable region comprising HCDR1, HCDR2 and HCDR3 with amino acid sequences as shown in SEQ ID NO: 28, SEQ ID NO: 74 and SEQ ID NO: 30, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XVII) a heavy chain variable region comprising HCDR1, HCDR2 and HCDR3 with amino acid sequences as shown in SEQ ID NO: 28, SEQ ID NO: 74 and SEQ ID NO: 75, respectively; and a light chain variable region comprising LCDR1, LCDR2 and LCDR3 with amino acid sequences as shown in SEQ ID NO: 13, SEQ ID NO: 14 and SEQ ID NO: 15, respectively.

In some embodiments, the amino acid sequence of the heavy chain variable region of the present invention comprises a sequence selected from SEQ ID NOs: 42, 45, 47, 49, 51, 53, 54, 76, 78, 82 and 83, or an amino acid sequence having at least 95%, 96%, 97%, 98%, or 99% identity to any of the sequences as shown in SEQ ID NOs: 42, 45, 47, 49, 51, 53, 54, 76, 78, 82 and 83; and
the amino acid sequence of the light chain variable region comprises a sequence selected from SEQ ID NOs: 43, 44, 46, 48, 50 and 52, or an amino acid sequence having at least 95%, 96%, 97%, 98%, or 99% identity to any of the sequences as shown in SEQ ID NOs: 43, 44, 46, 48, 50 and 52.

In some embodiments, the antibody or the antigen binding fragment thereof of the present invention comprises:
(I) a heavy chain variable region with an amino acid sequence as shown in SEQ ID NO: 42 and a light chain variable region with an amino acid sequence as shown in SEQ ID NO: 43; or
(II) a heavy chain variable region with an amino acid sequence as shown in SEQ ID NO: 42 and a light chain variable region with an amino acid sequence as shown in SEQ ID NO: 44; or
(III) a heavy chain variable region with an amino acid sequence as shown in SEQ ID NO: 45 and a light chain variable region with an amino acid sequence as shown in SEQ ID NO: 46; or
(IV) a heavy chain variable region with an amino acid sequence as shown in SEQ ID NO: 47 and a light chain variable region with an amino acid sequence as shown in SEQ ID NO: 48; or
(V) a heavy chain variable region with an amino acid sequence as shown in SEQ ID NO: 49 and a light chain variable region with an amino acid sequence as shown in SEQ ID NO: 50; or
(VI) a heavy chain variable region with an amino acid sequence as shown in SEQ ID NO: 51 and a light chain variable region with an amino acid sequence as shown in SEQ ID NO: 52; or
(VII) a heavy chain variable region with an amino acid sequence as shown in SEQ ID NO: 53 and a light chain variable region with an amino acid sequence as shown in SEQ ID NO: 50; or
(VIII) a heavy chain variable region with an amino acid sequence as shown in SEQ ID NO: 54 and a light chain variable region with an amino acid sequence as shown in SEQ ID NO: 52; or
(IX) a heavy chain variable region with an amino acid sequence as shown in SEQ ID NO: 45 and a light chain variable region with an amino acid sequence as shown in SEQ ID NO: 48; or
(X) a heavy chain variable region with an amino acid sequence as shown in SEQ ID NO: 51 and a light chain variable region with an amino acid sequence as shown in SEQ ID NO: 46; or
(XI) a heavy chain variable region with an amino acid sequence as shown in SEQ ID NO: 49 and a light chain variable region with an amino acid sequence as shown in SEQ ID NO: 46.

In some embodiments, the antibody or the antigen binding fragment thereof of the present invention comprises a heavy chain variable region and a light chain variable region, wherein
the amino acid sequence of the heavy chain variable region comprises a sequence selected from SEQ ID NOs: 55, 57, 58, 59, 60, 76, 78, 79, 82 and 83, or an amino acid sequence having at least 95%, 96%, 97%, 98%, or 99% identity to any of the sequences as shown in SEQ ID NOs: 55, 57, 58, 59, 60, 76, 78, 79, 82 and 83; and
the amino acid sequence of the light chain variable region comprises a sequence selected from SEQ ID NOs: 56, 61, 77, 80 and 81, or an amino acid sequence having at least 95%, 96%, 97%, 98%, or 99% identity to the sequences as shown in SEQ ID NOs: 56, 61, 77, 80 and 81.

In some embodiments, the antibody or the antigen binding fragment thereof of the present invention comprises a heavy chain variable region and a light chain variable region, wherein the heavy chain variable region comprises an amino acid sequence as shown in any one of SEQ ID NO: 55, 57, 60, 76, 78, 79, 82 or 83; and the light chain variable region comprises an amino acid sequence as shown in any one of SEQ ID NO: 56, 61, 77, 80 or 81.

In some embodiments, the antibody or the antigen binding fragment thereof of the present invention comprises a heavy chain variable region and a light chain variable region, wherein the heavy chain variable region comprises an amino acid sequence as shown in SEQ ID NO: 55, 57, 82 or 83; and the light chain variable region comprises an amino acid sequence as shown in SEQ ID NO: 56.

In some embodiments, the antibody or the antigen binding fragment thereof of the present invention comprises a heavy chain variable region and a light chain variable region, wherein the heavy chain variable region comprises an amino acid sequence as shown in SEQ ID NO: 60, 76 or 78; and the light chain variable region comprises an amino acid sequence as shown in SEQ ID NO: 61.

In some embodiments, the antibody or the antigen binding fragment thereof of the present invention comprises a heavy chain variable region and a light chain variable region, wherein
the heavy chain variable region comprises an amino acid sequence as shown in SEQ ID NO: 55; and the light chain variable region comprises an amino acid sequence as shown in SEQ ID NO: 56; or
the heavy chain variable region comprises an amino acid sequence as shown in SEQ ID NO: 57; and the light chain variable region comprises an amino acid sequence as shown in SEQ ID NO: 56; or
the heavy chain variable region comprises an amino acid sequence as shown in SEQ ID NO: 58; and the light chain variable region comprises an amino acid sequence as shown in SEQ ID NO: 56; or
the heavy chain variable region comprises an amino acid sequence as shown in SEQ ID NO: 59; and the light chain variable region comprises an amino acid sequence as shown in SEQ ID NO: 56; or
the heavy chain variable region comprises an amino acid sequence as shown in SEQ ID NO: 60; and the light chain variable region comprises an amino acid sequence as shown in SEQ ID NO: 61; or
the heavy chain variable region comprises an amino acid sequence as shown in SEQ ID NO: 76; and the light chain variable region comprises an amino acid sequence as shown in SEQ ID NO: 77; or
the heavy chain variable region comprises an amino acid sequence as shown in SEQ ID NO: 79; and the light chain variable region comprises an amino acid sequence as shown in SEQ ID NO: 80; or
the heavy chain variable region comprises an amino acid sequence as shown in SEQ ID NO: 55; and the light chain variable region comprises an amino acid sequence as shown in SEQ ID NO: 81. In some embodiments, the antibody or the antigen binding fragment thereof of the present invention comprises: a heavy chain having an amino acid sequence as shown in SEQ ID NO: 64 or a variant thereof or SEQ ID NO: 68 or a variant thereof, and a light chain having an amino acid sequence as shown in SEQ ID NO: 65 or a variant thereof or SEQ ID NO: 69 or a variant thereof, wherein the variant comprises 1, 2, 3, 4 or 5 amino acid changes in its variable region;
preferably, the variant of SEQ ID NO: 64 comprises an amino acid change at position 50 or 63 or a combination thereof; and/or the variant of SEQ ID NO: 68 comprises an amino acid change at position 63 or 65 or a combination thereof;
preferably, the variant of SEQ ID NO: 64 comprises S50C or S63T or a combination thereof; and/or the variant of SEQ ID NO: 68 comprises G63D or T65K or a combination thereof.

In some embodiments, the antibody or the antigen binding fragment thereof of the present invention comprises: a heavy and a light chain, wherein
the heavy chain comprises an amino acid sequence as shown in SEQ ID NO: 64, or an amino acid sequence having at least 90%, 92%, 94%, 95%, 96%, 97%, 98% or 99% sequence identity to the amino acid sequence as shown in SEQ ID NO: 64; and the light chain comprises an amino acid sequence as shown in SEQ ID NO: 65, or an amino acid sequence having at least 90%, 92%, 94%, 95%, 96%, 97%, 98% or 99% sequence identity to the amino acid sequence as shown in SEQ ID NO: 65; or
the heavy chain comprises an amino acid sequence as shown in SEQ ID NO: 68, or an amino acid sequence having at least 90%, 92%, 94%, 95%, 96%, 97%, 98% or 99% sequence identity to the amino acid sequence as shown in SEQ ID NO: 68; and the light chain comprises an amino acid sequence as shown in SEQ ID NO: 69, or an amino acid sequence having at least 90%, 92%, 94%, 95%, 96%, 97%, 98% or 99% sequence identity to the amino acid sequence as shown in SEQ ID NO: 69; or
the heavy chain comprises an amino acid sequence as shown in SEQ ID NO: 84, or an amino acid sequence having at least 90%, 92%, 94%, 95%, 96%, 97%, 98% or 99% sequence identity to the amino acid sequence as shown in SEQ ID NO: 84; and the light chain comprises an amino acid sequence as shown in SEQ ID NO: 85, or an amino acid sequence having at least 90%, 92%, 94%, 95%, 96%, 97%, 98% or 99% sequence identity to the amino acid sequence as shown in SEQ ID NO: 85; or
the heavy chain comprises an amino acid sequence as shown in SEQ ID NO: 86, or an amino acid sequence having at least 90%, 92%, 94%, 95%, 96%, 97%, 98% or 99% sequence identity to the amino acid sequence as shown in SEQ ID NO: 86; and the light chain comprises an amino acid sequence as shown in SEQ ID NO: 87, or an amino acid sequence having at least 90%, 92%, 94%, 95%, 96%, 97%, 98% or 99% sequence identity to the amino acid sequence as shown in SEQ ID NO: 87; or
the heavy chain comprises an amino acid sequence as shown in SEQ ID NO: 88, or an amino acid sequence having at least 90%, 92%, 94%, 95%, 96%, 97%, 98% or 99% sequence identity to the amino acid sequence as shown in SEQ ID NO: 88; and the light chain comprises an amino acid sequence as shown in SEQ ID NO: 89, or an amino acid sequence having at least 90%, 92%, 94%, 95%, 96%, 97%, 98% or 99% sequence identity to the amino acid sequence as shown in SEQ ID NO: 89; or
the heavy chain comprises an amino acid sequence as shown in SEQ ID NO: 90, or an amino acid sequence having at least 90%, 92%, 94%, 95%, 96%, 97%, 98% or 99% sequence identity to the amino acid sequence as shown in SEQ ID NO: 90; and the light chain comprises an amino acid sequence as shown in SEQ ID NO: 91, or an amino acid sequence having at least 90%, 92%, 94%, 95%, 96%, 97%, 98% or 99% sequence identity to the amino acid sequence as shown in SEQ ID NO: 91; or
the heavy chain comprises an amino acid sequence as shown in SEQ ID NO: 92, or an amino acid sequence having at least 90%, 92%, 94%, 95%, 96%, 97%, 98% or 99% sequence identity to the amino acid sequence as shown in SEQ ID NO: 92; and the light chain comprises an amino acid sequence as shown in SEQ ID NO: 93, or an amino acid sequence having at least 90%, 92%, 94%, 95%, 96%, 97%, 98% or 99% sequence identity to the amino acid sequence as shown in SEQ ID NO: 93; or
the heavy chain comprises an amino acid sequence as shown in SEQ ID NO: 94, or an amino acid sequence having at least 90%, 92%, 94%, 95%, 96%, 97%, 98% or 99% sequence identity to the amino acid sequence as shown in SEQ ID NO: 94; and the light chain comprises an amino acid sequence as shown in SEQ ID NO: 95, or an amino acid sequence having at least 90%, 92%, 94%, 95%, 96%, 97%, 98% or 99% sequence identity to the amino acid sequence as shown in SEQ ID NO: 95; or
the heavy chain comprises an amino acid sequence as shown in SEQ ID NO: 96, or an amino acid sequence having at least 90%, 92%, 94%, 95%, 96%, 97%, 98% or 99% sequence identity to the amino acid sequence as shown in SEQ ID NO: 96; and the light chain comprises an amino acid sequence as shown in SEQ ID NO: 97, or an amino acid sequence having at least 90%, 92%, 94%, 95%, 96%, 97%, 98% or 99% sequence identity to the amino acid sequence as shown in SEQ ID NO: 97; or
the heavy chain comprises an amino acid sequence as shown in SEQ ID NO: 98, or an amino acid sequence having at least 90%, 92%, 94%, 95%, 96%, 97%, 98% or 99% sequence identity to the amino acid sequence as shown in SEQ ID NO: 98; and the light chain comprises an amino acid sequence as shown in SEQ ID NO: 99, or an amino acid sequence having at least 90%, 92%, 94%, 95%, 96%, 97%, 98% or 99% sequence identity to the amino acid sequence as shown in SEQ ID NO: 99; or
the heavy chain comprises an amino acid sequence as shown in SEQ ID NO: 100, or an amino acid sequence having at least 90%, 92%, 94%, 95%, 96%, 97%, 98% or 99% sequence identity to the amino acid sequence as shown in SEQ ID NO: 100; and the light chain comprises an amino acid sequence as shown in SEQ ID NO: 101, or an amino acid sequence having at least 90%, 92%, 94%, 95%, 96%, 97%, 98% or 99% sequence identity to the amino acid sequence as shown in SEQ ID NO: 101.

In some embodiments, the antibody or the antigen binding fragment thereof of the present invention comprises:
(I) a heavy chain with an amino acid sequence as shown in SEQ ID NO: 64 and a light chain with an amino acid sequence as shown in SEQ ID NO: 65; or
(II) a heavy chain with an amino acid sequence as shown in SEQ ID NO: 68 and a light chain with an amino acid sequence as shown in SEQ ID NO: 69.

In some embodiments, the antibody of the present invention is a murine antibody, a chimeric antibody, a humanized antibody, or a fully human antibody.

In some embodiments, the antigen binding fragment of the present invention is an Fab, an Fab', an Fab'-SH, an F(ab')2, an Fv, an scFv or an sdAb or a diabody.

In some embodiments, the antibody of the present invention is of any IgG subtype, such as IgG1, IgG2, IgG3 or IgG4; Preferably, the antibody is hypofucosylated or afucosylated.

In some embodiments, the antibody of the present invention is hypofucosylated.

In some embodiments, the antibody of the present invention is afucosylated.

In another aspect, the present invention provides an isolated anti-CLDN-18.2 antibody or antigen binding fragment thereof having one or more of the following properties:
(1) binding to the same or completely or partially overlapping epitope of human CLDN-18.2 protein as the anti-CLDN-18.2 antibody or the antigen binding fragments thereof described herein;
(2) competing with the anti-CLDN-18.2 antibody or the antigen binding fragment thereof described herein to bind to an epitope of human CLDN-18.2 protein;
(3) binding to human CLDN-18.2 protein, but not to human CLDN-18.1 protein;
(4) inducing the ADCC effect of cells expressing human CLDN-18.2 protein; and
(5) inducing the CDC effect of cells expressing human CLDN-18.2 protein.

In yet another aspect, the present invention provides a polynucleotide coding the anti-CLDN-18.2 antibody or the antigen binding fragment thereof as described herein.

In yet another aspect, the present invention provides an expression vector comprising the polynucleotide as described herein, preferably, the vector is a eukaryotic expression vector.

In yet another aspect, the present invention provides a host cell comprising the polynucleotide as described herein or the expression vector as described herein, or expressing the anti-CLDN-18.2 antibody or the antigen binding fragment thereof as described herein, preferably, the host cell is a eukaryotic cell, more preferably a mammalian cell.

In yet another aspect, the present invention provides a method for preparing the anti-CLDN-18.2 antibody or the antigen binding fragment thereof as described herein, the method comprises culturing the host cell as describe herein under conditions suitable for expression of the antibody or the antigen binding fragment thereof, and recovering the expressed antibody or the antigen binding fragment thereof from the host cell.

In yet another aspect, the present invention provides a pharmaceutical composition comprising the anti-CLDN-18.2 antibody or the antigen binding fragment thereof as described herein, the polynucleotide as described herein, the expression vector as described herein, or the host cell as described herein, and a pharmaceutically acceptable carrier or excipient.

In yet another aspect, the present invention provides use of the antibody or the antigen binding fragment thereof as described herein, the polynucleotide as described herein, the expression vector as described herein, the host cell as described herein, or the pharmaceutical composition as described herein in the preparation of a medicament for the treatment and/or prevention of a disease or condition mediated by CLDN-18.2; preferably, the disease or condition is cancer.

In yet another aspect, the present invention provides the antibody or the antigen binding fragment thereof as described herein, the polynucleotide as described herein, the expression vector as described herein, the host cell as described herein, or the pharmaceutical composition as described herein for the treatment and/or prevention of a disease or condition mediated by CLDN-18.2, preferably, the disease or condition is cancer.

In yet another aspect, the present invention provides a method of treating and/or preventing a disease or condition mediated by CLDN-18.2, comprising administering to a subject in need thereof the antibody or the antigen binding fragment thereof as described herein, the polynucleotide as described herein, the expression vector as described herein, the host cell as described herein, or the pharmaceutical composition as described herein; preferably, the disease or condition is cancer.

In some embodiments, the cancer is selected from gastric cancer, esophageal cancer, gastroesophageal cancer, pancreatic cancer, cholangiocarcinoma, lung cancer, ovarian cancer, colon cancer, liver cancer, head and neck cancer, gallbladder cancer, intestinal cancer, and bladder cancer.

In yet another aspect, the present invention provides a pharmaceutical combination comprising the antibody or the antigen binding fragment thereof as described herein, the polynucleotide as described herein, the expression vector as described herein, the host cell as described herein, or the pharmaceutical composition as described herein, and one or more additional therapeutic agents.

In yet another aspect, the present invention provides a kit comprising the antibody or the antigen binding fragment thereof as described herein, the polynucleotide as described herein, the expression vector as described herein, the host cell as described herein, or the pharmaceutical composition as described herein; preferably, the kit further comprises an administration device.

In yet another aspect, the present invention provides a method for detecting the presence of CLDN-18.2 in a sample using the antibody or the antigen binding fragment thereof as described herein.

### Brief Description of the Drawings

Fig. 1: cell-level affinity of chimeric anti-CLDN-18.2 antibody determined by flow cytometry.
Figs. 2a and 2b: ADCC activity of chimeric anti-CLDN-18.2 antibody determined by reporter gene method.
Fig. 3: CDC activity of chimeric anti-CLDN-18.2 antibody determined by flow cytometry.
Fig. 4a, 4b and 4c: cell-level affinity of humanized anti-CLDN-18.2 antibody determined by flow cytometry.
Fig. 5a, 5b and 5c: ADCC activity of humanized anti-CLDN-18.2 antibody determined by reporter gene method.
Fig. 6a, 6b and 6c: CDC activity of humanized anti-CLDN-18.2 antibody determined by flow cytometry.
Fig. 7: inhibitory effect of humanized anti-CLDN-18.2 antibody on tumour growth of human gastric cancer MKN45 hClaudin18.2 Mixeno transplanted in M-NSG mice.
Fig. 8: inhibitory effect of humanized anti-CLDN-18.2 antibody on tumour growth of human pancreatic cancer hCLDN18.2 MIA PaCa-2 transplanted in CB-17 SCID mice.

### Detailed Description of Embodiments

### Definitions

The practice of the present invention may employ, unless otherwise indicated, conventional techniques of molecular biology (including recombinant techniques), microbiology, cell biology, biochemistry, and immunology, which are within the skill of the art.

In order to understand the present invention more easily, certain technical terms are specifically defined as follows. Unless otherwise explicitly defined elsewhere herein, all technical and scientific terms used herein have the meanings commonly understood by those of ordinary skill in the art to which the present invention belongs. For definitions and terms in this field, professionals can refer to Current Protocols in Molecular Biology (Ausubel). Abbreviations for amino acid residues are the standard 3-letter and/or 1-letter codes used in the art to refer to one of the 20 commonly used L-amino acids. As used herein (including the claims), singular forms include their corresponding plural forms unless the context explicitly dictates otherwise.

The term "about" when used in connection with a numerical value is meant to encompass numerical values within a range having a lower limit that is 5% smaller than the specified numerical value and an upper limit that is 5% larger than the specified numerical value.

The term "and/or" should be understood as meaning any one of the optional items or a combination of any two or more of the optional items.

The term "CLDN-18.2" or "Claudin18.2", is one of the two splicing variants of Claudin 18. The term refers to any native CLDN-18.2 from any vertebrate, including mammals such as primates (for example, human), and rodents (for example, mice and rats), unless otherwise stated. The term encompasses "full-length" unprocessed CLDN-18.2 as well as any form of CLDN-18.2 or any fragment thereof produced by intracellular processing. The term also includes naturally occurring variants of CLDN-18.2, for example, splicing variants or allelic variants. In a preferred embodiment, CLDN-18.2 refers to full-length CLDN-18.2 or a fragment thereof (such as a mature fragment thereof lacking a signal peptide) from human and cynomolgus monkey.

The term "percent (%) amino acid sequence identity" or simply "identity" is defined as the percentage of amino acid residues in a candidate amino acid sequence that are identical to the amino acid residues in the reference amino acid sequence, after aligning the amino acid sequences and introducing gaps, if necessary, to achieve the maximum percent sequence identity, and not considering any conservative substitutions as part of the sequence identity. Alignment of sequences for the purpose of determining percent amino acid sequence identity can be achieved by using a variety of methods in the art, for instance, using publicly available computer software, such as BLAST, BLAST-2, ALIGN or MEGALIGN (DNASTAR) software. A person skilled in the art can determine appropriate parameters for measuring alignment, including any algorithms needed to achieve maximal alignment over the full length of the sequences being compared.

The term "immune response" refers to the action of, for example, lymphocytes, antigen presenting cells, phagocytic cells, granulocytes, and soluble macromolecules produced by the above cells or the liver (including antibodies, cytokines, and complements), this action results in selective damage to, destruction of or elimination from the human body of invading pathogens, pathogen-infected cells or tissues, cancer cells or normal human cells or tissues in the case of autoimmune or pathological inflammation.

The term "signal transduction pathway" or "signal transduction activity" refers to a biochemical causal relationship, usually initiated by protein-protein interactions, such as the binding of a growth factor to a receptor; this relationship results in the transmission of a signal from one part of a cell to another part. Typically, transmission involves specific phosphorylation of one or more tyrosine, serine, or threonine residues on one or more proteins in a cascade of reactions leading to signal transduction. The penultimate process often involves nuclear events, resulting in changes in gene expression.

The terms "activity" or "biological activity", or the terms "biological property" or "biological characteristics" are used interchangeably herein, including but not limited to epitope/antigen affinity and specificity, ability to neutralize or antagonize CLDN-18.2 activity *in vivo* or *in vitro,* IC₅₀, *in vivo* stability of the antibody and immunogenic property of the antibody. Other identifiable biological properties or characteristics of antibodies known in the art include, for example, cross-reactivity (*i.e*., cross-reactive usually with a non-human homologue of a target peptide, or with other proteins or tissues.), and ability to maintain high expression level of proteins in mammalian cells. The aforementioned properties or characteristics are observed, measured or evaluated using techniques known in the art including, but not limited to, ELISA, FACS or BIACORE plasma resonance analysis, unrestricted *in vitro* or *in vivo* neutralization assays, receptor binding, cytokine or growth factor production and/or secretion, signal transduction and immunohistochemistry of tissue sections of different origins, including human, primate or any other origins.

The term "antibody" refers to any form of antibody that possesses the desired biological activity. Accordingly, it is used in the broadest sense and specifically includes, but is not limited to, monoclonal antibody (including full length monoclonal antibody), polyclonal antibody, multispecific antibody(such as bispecific antibody), humanized antibody, fully human antibody, chimeric antibody and camelized single domain antibody.

The term "isolated antibody" refers to the purified state of a binding compound, and in this context means that the molecule is substantially free of other biomolecules such as nucleic acids, proteins, lipids, sugars or other substances such as cell debris and growth media. The term "isolate (isolated)" does not imply the complete absence of such substances or the absence of water, buffers or salts, unless they are present in an amount that significantly interferes with the experimental or therapeutic use of the binding compound described herein.

The term "monoclonal antibody" refers to an antibody obtained from a substantially homogeneous population of antibody, *i*.*e*., the individual antibodies making up the population are identical except for possible naturally occurring mutations that may be present in minor amounts. Monoclonal antibodies are highly specific and are directed against a single antigen epitope. In contrast, conventional (polyclonal) antibody preparations typically include a large number of antibodies against (or specific for) different epitopes. The modifier "monoclonal" indicates the characteristic of an antibody obtained from a substantially homogeneous population of antibody and should not be construed as requiring production of the antibody by any particular method.

The term "full length antibody" refers to an immunoglobulin molecule comprising four peptide chains as it occurs in nature: two heavy (H) chains (approximately 50-70 kDa in full length) and two light (L) chains (approximately 25 kDa in full length) connected to each other by disulfide bonds. Each heavy chain consists of a heavy chain variable region (abbreviated herein as VH) and a heavy chain constant region (abbreviated herein as CH). The heavy chain constant region consists of 3 domains (CH1, CH2 and CH3). Each light chain consists of a light chain variable region (abbreviated herein as VL) and a light chain constant region. The light chain constant region consists of one domain CL. The VH and VL regions can be further subdivided into highly variable complementarity determining regions (CDRs) and more conserved regions called framework regions (FRs) spaced by CDRs. Each VH or VL region consists of 3 CDRs and 4 FRs arranged from amino terminal to carboxyl terminal, in the following order: FR1, CDR1, FR2, CDR2, FR3, CDR3, FR4. The variable regions of the heavy and light chains contain binding domains that interact with antigens. The constant region of the antibody can mediate the binding of the immunoglobulin to a host tissue or factor (comprising various cells (e.g., effector cells) of the immune system and the first component of the classical complement system (Clq)).

The term "Fc region" is used to define the C-terminal region of an immunoglobulin heavy chain that contains at least a portion of the constant region. The term includes native sequence Fc regions and variant Fc regions. In one embodiment, the human IgG heavy chain Fc region extends from Cys226 or from Pro230 of the heavy chain to the carboxy-terminus. However, the C-terminal lysine (Lys447) of the Fc region may or may not be present. Unless otherwise indicated herein, the numbering of amino acid residues in the Fc region or constant region is according to the EU numbering system, also known as the EU index, as described in Kabat, E.A. et al., Sequences of Proteins of Immunological Interest, 5th edition, Public Health Service National Institutes of Health, Bethesda, MD (1991), NIH Publication 91-3242.

The term "antigen binding fragment" of an antibody ("parent antibody") includes a fragment or a derivative of an antibody, typically comprising at least a fragment of the antigen binding region or the variable region (for example, one or more CDRs) of the parent antibody, and the fragment or the derivative of the antibody retains at least some of the binding specificity of the parent antibody. Examples of antigen binding fragment include, but are not limited to, Fab, Fab', F(ab')₂ and Fv fragments; diabodies; linear antibodies; single-stranded antibody molecules, such as sc-Fv; and nanobodies and multispecific antibodies formed from antibody fragments. When the antigen binding activity of an antibody is expressed on a molar concentration basis, a binding fragment or a derivative typically retains at least 10% of its antigen binding activity. Preferably the binding fragment or the derivative retains at least 20%, 50%, 70%, 80%, 90%, 95% or 100% or more of the antigen binding affinity of the parent antibody. It is also contemplated that an antigen binding fragment of an antibody may include conservative or non-conservative amino acid substitutions that do not significantly change its biological activity (referred to as "conservative variants" or "functionally conservative variants" of the antibody). The term "binding compound" refers to both an antibody and a binding fragment thereof.

The term "single-chain Fv" or "scFv" antibody refers to an antibody fragment comprising the VH domain and the VL domain of the antibody, wherein these domains are present in a single polypeptide chain. Fv polypeptide generally further comprise a polypeptide linker between the VH domain and the VL domain, which enables the scFv to form the desired structure for antigen binding.

The term "domain antibody" is an immunologically functional immunoglobulin fragment containing only the variable region of a heavy chain or the variable region of a light chain. In certain instances, two or more VH regions are covalently linked with a peptide linker to form a bivalent domain antibody. The two VH regions of the bivalent domain antibody may target the same or different antigens.

The term "bivalent antibody" comprises two antigen binding sites. In some cases, the two binding sites have the same antigen specificity. However, the bivalent antibody can be bispecific.

The term "diabody" refers to a small antibody fragment with two antigen binding sites, the fragment comprises a heavy chain variable domain (VH) linked to a light chain variable domain (VL) in the same polypeptide chain (VH-VL or VL-VH). By using a linker that is too short to allow pairing between the two domains in the same chain, the domain is forced to pair with the complementary domain of another chain to create two antigen binding sites.

The term "chimeric antibody" is an antibody having a variable domain of a first antibody and a constant domain of a second antibody, wherein the first antibody and the second antibody are from different species. Typically, the variable domain is derived from an antibody of a rodent, *etc.* (a "parent antibody"), while the constant domain sequence is derived from a human antibody such that compared with the parental rodent antibody, the obtained chimeric antibody is less likely to induce an adverse immune response in human subjects.

The term "humanized antibody" refers to a form of an antibody that contains sequences from both human and non-human (for example, mouses and rats) antibody. In general, a humanized antibody comprises at least one, usually two, variable domains, wherein all or substantially all of the hypervariable loops correspond to those of a non-human immunoglobulin, and all or substantially all of the framework (FR) regions correspond to those of a human immunoglobulin sequence. A humanized antibody optionally can comprise at least a portion of a human immunoglobulin constant region (Fc).

The term "fully human antibody" refers to an antibody that comprises only human immunoglobulin protein sequences. If the fully human antibody is produced in mice, mouse cells or hybridomas derived from mouse cells, the fully human antibody may contain mouse sugar chains. Likewise, "mouse antibody" refers to an antibody comprising only mouse immunoglobulin sequences. Alternatively, if the fully human antibody is produced in rats, rat cells or hybridomas derived from rat cells, the fully human antibody may contain rat sugar chains. Likewise, "rat antibody" refers to an antibody that comprises only rat immunoglobulin sequences.

An "isotype" antibody refers to the class of antibody (for example, IgM, IgE, and IgG such as IgG1, IgG2 or IgG4) based on the heavy chain constant region genes. The isotype also includes modified forms of one of these classes, wherein modifications have been made to alter Fc function, for example to enhance or attenuate effector function or binding to Fc receptors.

The term "epitope" refers to an antigen region to which an antibody binds. The epitope can be formed from contiguous amino acids or non-contiguous amino acids juxtaposed by the tertiary folding of the protein.

"Affinity" or "binding affinity" refers to the intrinsic binding affinity that reflects the interaction between members of a binding pair. The affinity of a molecule X for its partner Y can generally be represented by the equilibrium dissociation constant (K_{D}), which is ratio of the dissociation rate constant (k_{dis}) to the association rate constant (kₒₙ). Affinity can be measured by common methods known in the art. One specific method for measuring affinity is the ForteBio Kinetic Binding Assay herein.

The term "not binding" to a protein or cell means not binding to the protein or cell, or not binding to the protein or cell with high affinity, *i.e.,* the K_{D} for binding to the protein or cell is 1.0 × 10⁻⁶ M or more, more preferably 1.0 × 10⁻⁵ M or more, more preferably 1.0 × 10⁻⁴ M or more, 1.0 × 10⁻³ M or more, and more preferably 1.0 × 10⁻² M or more.

The term "high affinity" for an IgG antibody means a K_{D} for an antigen of 1.0 × 10⁻⁶ M or less, preferably 5.0 × 10⁻⁸ M or less, more preferably 1.0 × 10⁻⁸ M or less, 5.0 × 10⁻⁹ M or less, and more preferably 1.0 × 10⁻⁹ M or less. "High affinity" binding may vary for other antibody subtypes. For example, "high affinity" binding of the IgM subtype means a K_{D} of 10⁻⁶ M or less, preferably 10⁻⁷ M or less, and more preferably 10⁻⁸ M or less.

The terms "antibody-dependent cellular cytotoxicity", "antibody-dependent cell-mediated cytotoxicity" or "ADCC" refer to cell-mediated immune defense in which immune system effector cells actively lyse target cells, such as cancer cells, which has their cell membrane surface antigens bound by antibodies, such as Claudin18.2 antibody.

The term "complement dependent cytotoxicity" or "CDC" refers to the effector functions of IgG and IgM antibodies which, when bound to surface antigens, initiate the canonical complement pathway, including formation of a membrane attack complex and lysis of target cells. The antibody of the present invention, when bound to Claudin 18.2, induces CDC on cancer cells.

The term "nucleic acid" or "polynucleotide" refers to deoxyribonucleic acid (DNA) or ribonucleic acid (RNA) and polymers thereof in single- or double-stranded form. Unless expressly limited, the term includes nucleic acids containing analogs of known natural nucleotides and having similar binding properties to the reference nucleic acid and metabolized in a manner similar to naturally occurring nucleotides (see U.S. pat. No.8,278,036 to Kariko et al.*,* which discloses an mRNA molecule in which uridine is replaced by pseudouridine, a method for synthesizing the mRNA molecule, and a method for delivering therapeutic proteins *in vivo*)*.* Unless otherwise indicated, a particular nucleic acid sequence also implicitly encompasses conservatively modified variants thereof (for example, degenerate codon substitutions), alleles, orthologs, SNPs, and complementary sequences as well as the sequences explicitly indicated. Specifically, degenerate codon substitutions can be achieved by generating sequences in which the third positions of one or more selected (or all) codons are replaced with mixed bases and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" means any recombinant polynucleotide molecule (such as a plasmid, cosmid, virus, autonomously replicating polynucleotide molecule, bacteriophage, or linear or circular single- or double-stranded DNA or RNA polynucleotide molecule), which is derived from any source, capable of integrating with the genome or replicating autonomously, constitutes a polynucleotide molecule in which one or more polynucleotide molecules have been functionally linked (*i.e.,* operably linked). A recombinant construct typically comprises a polynucleotide of the present invention operably linked to a transcription initiation regulatory sequence that direct transcription of the polynucleotide in a host cell. Both heterologous and non-heterologous (*i*.*e*., endogenous) promoters can be used to direct expression of the nucleic acids of the present invention.

"Vector" refers to any recombinant polynucleotide construct that can be used for the purpose of transformation (*i.e.,* introduction of a heterologous DNA into a host cell). One type of vector is a "plasmid", which refers to a circular double-stranded DNA loop into which additional DNA segments can be ligated. Another type of vector is a viral vector, and additional DNA segments can be ligated into the viral genome. Certain vectors are capable of autonomous replication in a host cell into which they are introduced (for example, bacterial vectors having a bacterial origin of replication and episomal mammalian vectors). After introduction into a host cell, other vectors (for example, non-episomal mammalian vectors) integrate into the genome of the host cell and thus replicate along with the genome of the host cell. In addition, certain vectors are capable of directing the expression of operably linked genes. Such vector is referred to as "expression vector" herein.

The term "expression vector" as used herein refers to a nucleic acid molecule capable of replicating and expressing a gene of interest upon transformation, transfection or transduction into a host cell. The expression vector contains one or more phenotypic selectable markers and an origin of replication to ensure maintenance of the vector and to provide amplification in the host if desired.

"Activation", "stimulation" and "treatment" for a cell or receptor can have the same meaning, for example, the cell or receptor is activated, stimulated or treated with a ligand, unless the context dictates otherwise or explicitly. "Ligand" includes natural and synthetic ligands such as cytokines, cytokine variants, analogs, muteins, and binding compounds derived from antibodies. "Ligand" also includes small molecules, such as peptidomimetics of cytokines and peptidomimetics of antibodies. "Activation" can refer to cellular activation regulated by internal mechanisms as well as external or environmental factors. "Response/reaction", such as response of a cell, tissue, organ, or organism, includes changes in biochemical or physiological behaviors (such as concentration, density, adhesion or migration, gene expression rate, or differentiation status within a biological compartment), where the changes are related to activation, stimulation or treatment, or to internal mechanisms such as genetic programming.

As used herein, the term "treat" or "treatment" of any disease or condition refers in one embodiment to ameliorating the disease or condition (*i*.*e*., slowing or arresting or reducing the progression of or at least one clinical symptom of the disease). In another embodiment, "treat" or "treatment" refers to alleviating or improving at least one physical parameter, including those physical parameters that may not be discernible by the patient. In another embodiment, "treat" or "treatment" refers to modulating a disease or condition physically (for example, stabilization of discernible symptoms), physiologically (for example, stabilization of physical parameters), or both. Unless explicitly described herein, methods for assessing treatment and/or prevention of a disease are generally known in the art.

"Subject" includes any human or non-human animal. The term "non-human animal" includes all vertebrates, for example, mammals and non-mammals, such as non-human primates, sheep, dogs, cats, horses, cows, chickens, amphibians, reptiles, and the like. As used herein, the term "cyno" or "cynomolgus monkey" refers to cynomolgus monkeys.

Administration "in combination with" one or more other therapeutic agents includes both simultaneous (co)administration and sequential administration in any order.

"Therapeutically effective amount", "therapeutically effective dose" and "effective amount" refers to the amount of the CLDN-18.2 antibodies or the antigen binding fragments of the present invention that can effectively prevent or improve the symptoms of one or more diseases or conditions or the development of such diseases or conditions when administered to cells, tissues or subjects alone or in combination with other therapeutic agents. A therapeutically effective dose also refers to an amount of an antibody or an antigen binding fragment thereof sufficient to result in an amelioration of symptoms, for example, an amount that treats, cures, prevents or ameliorates an associated medical condition or increases the rate of treatment, cure, prevention or amelioration of such a condition. When the active ingredient is administered alone to a subject, the therapeutically effective dose refers to that ingredient only. When administered in combination, the therapeutically effective dose refers to the combined amount of the active ingredients which produces the therapeutic effect, whether in combination, sequentially or simultaneously. An effective amount of a therapeutic agent will result in an improvement in diagnostic criteria or parameters of at least 10%; usually at least 20%; preferably at least about 30%; more preferably at least 40%, and most preferably at least 50%.

"Cancer" and "cancerous" refer to or describe the physiological illness in mammals that is often characterized by unregulated cell growth. Included in this definition are benign and malignant cancers as well as dormant tumours or micrometastases. Examples of cancers include, but are not limited to, carcinoma, lymphoma, blastoma, sarcoma, and leukemia. More specific examples of such cancers include squamous cell carcinoma, lung cancer (including small cell lung cancer, non-small cell lung cancer, adenocarcinoma of the lung, and squamous cell carcinoma of the lung), peritoneal carcinoma, hepatocellular carcinoma, cancer of the stomach or gastric cancer (including gastrointestinal cancer), pancreatic cancer, glioblastoma, cervical cancer, ovarian cancer, liver cancer, bladder cancer, hepatoma, breast cancer, colon cancer, colorectal cancer, endometrial cancer or uterine cancer, salivary gland cancer, renal cancer or cancer of the kidney, hepatic cancer, prostate cancer, vulvar cancer, thyroid cancer, cancer of the liver, and various types of head and neck cancer, and B-cell lymphoma (including low-grade/follicular non-Hodgkin King's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small non-cleaved cell NHL, bulky disease NHL, mantle cell lymphoma, AIDS-related lymphoma, and Waldenstrom's macroglobulinemia), chronic lymphocytic leukemia (CLL), acute lymphoblastic leukemia (ALL), hairy cell leukemia, chronic myeloblastic leukemia, and post-transplant lymphoproliferative disorder (PTLD), as well as abnormal vascular proliferation associated with phakomatoses, edema (such as those associated with brain tumours) and Meigs's syndrome.

### Anti-CLDN-18.2 antibody

In one aspect, the present invention provides an anti-CLDN-18.2 antibody or an antigen binding fragment thereof. The term "anti-CLDN-18.2 antibody", "anti-CLDN-18.2", "CLDN-18.2 antibody" or "antibody that binds to CLDN-18.2" means that it can bind to CLDN-18.2 protein or fragment thereof with sufficient affinity so that the antibody can be used as a diagnostic and/or therapeutic agent targeting CLDN-18.2.

The antibody of the present invention can be produced using any suitable method for producing antibodies. Any suitable form of CLDN-18.2 can be used as an immunogen (antigen) for antibody production. By way of example and not limitation, any CLDN-18.2 variant or fragment thereof can be used as an immunogen. In some embodiments, hybridoma cells producing murine monoclonal anti-human CLDN-18.2 antibody can be produced by methods known in the art. The antibody derived from rodents (for example, mice) may cause unwanted antibody immunogenicity when used *in vivo* as a therapeutic drug, and repeated use leads to the immune response of the human body against therapeutic antibodies, and such immune response at least leads to the loss of therapeutic efficacy, and in severe cases leads to potentially fatal allergic reactions. One approach to reducing the immunogenicity of rodent antibodies includes the generation of chimeric antibodies, in which mouse variable regions are fused to human constant regions (Liu et al. (1987) Proc. Natl. Acad. Sci. USA 84:3439- 43). However, retention of intact rodent variable regions in the chimeric antibodies may still cause deleterious immunogenicity in patients. Grafting of complementarity determining region (CDR) loops of rodent variable domains onto human frameworks (*i*.*e*., humanization) has been used to further minimize rodent sequences (Jones et al. (1986) Nature 321:522; Verhoeyen et al. (1988) Science 239:1534).

In some embodiments, the chimeric or humanized antibody of the present invention can be prepared based on the sequence of the prepared murine monoclonal hybridoma antibody. DNA coding the heavy and light chains of an immunoglobulin can be obtained from murine hybridomas of interest and engineered to contain non-murine (for example, human) immunoglobulin sequences using standard molecular biology techniques.

In some embodiments, the chimeric CLDN-18.2 antibody of the present invention can be prepared by effectively linking the variable regions of immunoglobulin heavy chain and light chain from hybridoma with the constant region of human IgG (see, for example, U.S. Pat. No.4,816,567 belonging to Cabilly et al.) by methods known in the art to obtain a chimeric heavy chain and a chimeric light chain. In some embodiments, the constant region comprised in the chimeric antibody of the present invention can be selected from any human IgG subtype, such as IgG1, IgG2, IgG3, IgG4, preferably IgG4.

In some embodiments, the chimeric CLDN-18.2 antibody of the present invention can be obtained by transfecting expression cells with an expression plasmid of the chimeric light chain and the chimeric heavy chain in a manner of "mix and match", and the above binding assay and other conventional binding assays (for example, ELISA) can be used to determine the CLDN-18.2 binding of such "mixed and matched" antibody.

The precise amino acid sequence boundaries of the variable region CDRs of the antibody of the present invention can be determined using any of a number of well-known schemes, including Chothia based on the three-dimensional structure of an antibody and the topology of a CDR loop (Chothia et al. (1989) Nature 342:877-883; Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th ed., U.S. Department of Health and Human Services, National Institutes of Health (1987)), AbM (University of Bath), Contact (University College London), International ImMunoGeneTics database (IMGT) (1999 Nucleic Acids Research, 27, 209-212), and the North CDR definition based on affinity propagation clustering using a large number of crystal structures. The boundary of the CDRs of the antibody of the present invention may be determined by a person skilled in the art according to any scheme in the art (for example different assignment systems or combinations).

It should be noted that the boundaries of the CDRs of the variable regions of the same antibody obtained based on different assignment systems may be different. That is, the CDR sequences of the variable regions of the same antibody defined under different assignment systems are different. Therefore, when it comes to defining an antibody with specific CDR sequences defined in the present invention, the scope of the antibody also encompasses such an antibody whose variable region sequences comprise the specific CDR sequences but whose claimed CDR boundaries are different from the specific CDR boundaries defined by the present invention due to the application of a different scheme (for example, different assignment systems or combinations).

Antibodies with different specificities (*i.e.,* different binding sites for different antigens) have different CDRs. However, although CDRs vary from antibody to antibody, only a limited number of amino acid positions within a CDR are directly involved in antigen binding. Using at least two of the methods of Kabat, Chothia, AbM, Contact and North, a minimum overlapping region can be determined, thereby providing a "minimum binding unit" for antigen binding. The minimal binding unit may be a subpart of a CDR. As will be apparent to those skilled in the art, the residues of the remainder of the CDR sequences can be determined from the structure and protein folding of the antibody. Accordingly, the present invention also contemplates variants of any of the CDRs presented herein. For example, in a variant of a CDR, the amino acid residues of the minimum binding unit can remain unchanged, while the remaining CDR residues defined according to Kabat or Chothia can be replaced by conserved amino acid residues.

For the humanized antibody of the present invention, the murine CDR regions can be inserted into the human germline framework regions using methods known in the art. See U.S. Patent No. 5,225,539 to Winter et al. and U.S. Patent No. 5,530,101; 5,585,089; 5,693,762 and 6,180,370 to Queen et al.

In some embodiments, amino acid changes include amino acid deletions, insertions or substitutions. In some embodiments, the anti-CLDN-18.2 antibodies or the antigen binding fragments thereof of the present invention include those antibodies that have amino acid sequences have been mutated by amino acid deletion, insertion or substitution, but still have at least about 90%, 91%, 92%, 93%, 94%, 95%, 96%, 97%, 98%, 99% or 100% identity to the above antibodies (especially in the CDR regions depicted in the above sequences). In some embodiments, the antibody of the present invention has no more than 1, 2, 3, 4 or 5 amino acid mutations in the CDR region by amino acid deletion, insertion or substitution when compared to the CDR region depicted in the specific sequence.

In some embodiments, the polynucleotide coding the antibody of the present invention include a polynucleotide that have been mutated by nucleotide deletion, insertion, or substitution but still have at least about 60, 70, 80, 90, 95, or 100% identity to the coding region corresponding to the CDR depicted in the sequence described above.

In some embodiments, one or more amino acid modifications can be introduced into the Fc region of the antibody provided herein to generate a Fc region variant. The Fc region variant may comprise human Fc region sequences (for example, human IgG1, IgG2, IgG3 or IgG4 Fc regions) comprising amino acid modifications (for example, substitutions) at one or more amino acid positions.

In some embodiments, it may be desirable to generate a cysteine-engineered antibody, such as "thioMAb," in which one or more residues of the antibody are replaced with cysteine residues.

In some embodiments, an antibody can be altered to increase or decrease its degree of glycosylation and/or alter its glycosylation pattern. Addition or deletion of glycosylation sites to an antibody is conveniently accomplished by altering the amino acid sequence to create or remove one or more glycosylation sites. For example, one or more amino acid substitutions can be made to eliminate one or more glycosylation sites, thereby eliminating glycosylation at that site. The antibody can be prepared with altered types of glycosylation, for example, a hypofucosylated antibody with reduced amount of fucosyl residues or an afucosylated antibody or an antibody with increased bisected GlcNac structures. Such altered glycosylation patterns have been shown to increase the ADCC ability of an antibody.

In some preferred embodiments, the present invention provides an antibody that is hypofucosylated or afucosylated such that the binding affinity of the antibody to receptors expressed on effector cells can be significantly increased, thereby resulting in the antibody having enhanced antibody-dependent cell-mediated cytotoxicity (ADCC) activity. The average value of fucose in the sugar chain at Asn 297 can be calculated relative to the sum of all sugar structures (such as complex, hybrid and high mannose structures) linked to Asn 297 by MALDI-TOF mass spectrometry, thereby determining the amount of fucose, for example as described in WO 2008/077546. Asn297 refers to the aspartic acid residue located at approximately position 297 (EU numbering of Fc region residues) in the Fc region; however, due to minor sequence variations in the antibody, Asn297 may also be located approximately ± 3 amino acid positions upstream or downstream of position 297, *i.e.,* between position 294 and position 300. See, for example, US 2003/0157108; US 2004/0093621. Such antibody variants can be produced in cell lines capable of producing defucosylated or hypofucosylated antibodies. Examples of such cells include Lecl3 CHO cells deficient in protein fucosylation (Ripka, J. et al., Arch. Biochem. Biophys. 249 (1986): 533-545; US 2003/0157108). In some embodiments, fucose residues of the antibody are cleaved using fucosidase. In some embodiments, glycoform modulators are used to manipulate the fucose residues of the antibody. The glycoform modulators may be CDFS01, which is commercially available from Shanghai OPM Biosciences Co., Ltd. Defucosylation can be performed by conventional methods known in the art.

The level of fucosylation of an antibody can be structurally defined. As used herein, "non-fucosylation" or "afucosylation" means that in the antibody, the content of fucose is less than 5%, such as about 0%, less than 1%, less than 2%, less than 3%, and less than 4%. The term "hypofucosylation" means that in the antibody, the content of fucose is about greater than or equal to 5% and less than 30%; for example, the content of fucose in the antibody is about 5%-10%, 10%-15%, 15%-20%, 20%-25%, 25%-30%, 10%-20%, 20%-30 %, and 10%-30%. The term "hypofucosylation or afucosylation" means that the content of fucose in the antibody is less than 30%.

In some embodiments, the antibody provided herein can be further modified to contain other non-protein moieties known and readily available in the art. Moieties suitable for antibody derivatization include, but are not limited to, water soluble polymers. Non-limiting examples of water soluble polymers include, but are not limited to, polyethylene glycol (PEG), ethylene glycol/propylene glycol copolymer, carboxymethylcellulose, dextran, polyvinyl alcohol, polyvinylpyrrolidone, poly-1,3-dioxane, poly-1,3,6-trioxane, ethylene/maleic anhydride copolymer, polyamino acid (homopolymer or random copolymer), and dextran or poly(n-vinyl pyrrolidone) polyethylene glycol, propylene glycol homopolymer, polypropylene oxide/ethylene oxide copolymer, polyoxyethylated polyols (such as glycerin), polyvinyl alcohol, and mixtures thereof.

### Antibody expression

In yet another aspect, the present invention provides a polynucleotide coding the anti-CLDN-18.2 antibody or the antigen binding fragment thereof as described herein. The polynucleotide may comprise a polynucleotide coding the amino acid sequence of the light chain variable region and/or the heavy chain variable region of the antibody, or comprise a polynucleotide coding the amino acid sequence of the light chain and/or the heavy chain of the antibody.

In yet another aspect, the present invention provides an expression vector comprising the polynucleotide as described herein, preferably, the vector is a eukaryotic expression vector. In some embodiments, the polynucleotide as described herein is contained in one or more expression vectors.

In yet another aspect, the present invention provides a host cell comprising the polynucleotide as described herein or the expression vector as described herein, preferably, the host cell is a eukaryotic cell, more preferably a mammalian cell.

In yet another aspect, the present invention provides a method for preparing the anti-CLDN-18.2 antibody or the antigen binding fragment thereof as described herein, the method comprises expressing the antibody or the antigen binding fragment thereof in the host cell as described herein under conditions suitable for expression of the antibody or the antigen binding fragment thereof, and recovering the expressed antibody or the antigen binding fragment thereof from the host cell.

The prensent invention provides mammalian host cells for expressing the recombinant antibody of the present invention, including the many immortalized cell lines available from the American Type Culture Collection (ATCC). These include, *inter alia,* Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells and many others cell line. The mammalian host cells include human, mouse, rat, dog, monkey, pig, goat, bovine, horse and hamster cells. Particularly preferred cell lines are selected by determining which cell lines have high expression levels.

In one embodiment, the present invention provides a method for preparing an anti-CLDN-18.2 antibody, wherein the method comprises that an expression vector is introduced into a mammalian host cell, and the host cell is cultured for a sufficient period of time to allow the antibody to be expressed in the host cell, or more preferably to allow the antibody to be secreted into the culture medium where the host cell grows, thereby producing the antibody. The antibody can be recovered from the culture medium using standard protein purification methods.

It is likely that antibodies expressed by different cell lines or expressed in transgenic animals have different glycosylation from each other. However, all the antibodies coded by the nucleic acid molecules provided herein or comprising the amino acid sequences provided herein are part of the present invention, regardless of the glycosylation of the antibodies. Also, in certain embodiments, non-fucosylated antibodies are advantageous because they generally have stronger efficacy than their fucosylated counterparts *in vitro* and *in vivo* and are less likely to be immunogenic, because their sugar structure is a normal component of natural human serum IgG.

### Pharmaceutical composition and pharmaceutical preparation

In yet another aspect, the present invention provides a pharmaceutical composition comprising the anti-CLDN-18.2 antibody or the antigen binding fragment thereof as described herein, the polynucleotide as described herein, the expression vector as described herein, the host cell as described herein, and a pharmaceutically acceptable carrier or excipient.

It should be understood that the anti-CLDN-18.2 antibody or the pharmaceutical composition thereof provided by the present invention can be administered in combination with appropriate carriers, excipients and other agents in the preparation, so as to provide improved transfer, delivery, tolerance and the like.

The term "pharmaceutical composition" refers to a preparation that allows the active ingredients contained therein to be present in a biologically effective form and does not contain additional ingredients that would be unacceptably toxic to the subject to which the preparation is administered.

The anti-CLDN-18.2 antibody of the present invention having the desired purity can be mixed with one or more optional pharmaceutical excipients (Remington's Pharmaceutical Sciences, 16th edition, Osol, A. ed. (1980)) to prepare the pharmaceutical preparation comprising the anti-CLDN-18.2 antibody as described herein, preferably in the form of an aqueous solution or a lyophilized preparation.

The pharmaceutical composition or preparation of the present invention may also comprise one or more other active ingredients as required for the particular indication being treated, preferably those active ingredients with complementary activities that do not adversely affect each other. In some embodiments, other active ingredients are chemotherapeutic agents, immune checkpoint inhibitors, growth inhibitors, antibiotics, or various known anti-tumour or anti-cancer agents, and the active ingredients are present in a suitable combination in an amount effective for the intended use. In some embodiments, the pharmaceutical composition of the present invention further comprises a composition of a polynucleotide coding the anti-CLDN-18.2 antibody.

In yet another aspect, the present invention provides a pharmaceutical combination comprising the antibody or the antigen binding fragment thereof as described herein, the polynucleotide as described herein, the expression vector as described herein, the host cell as described herein, or the pharmaceutical composition as described herein, and one or more additional therapeutic agents.

In yet another aspect, the present invention provides a kit comprising the antibody or the antigen binding fragment thereof as described herein, the polynucleotide as described herein, the expression vector as described herein, the host cell as described herein, or the pharmaceutical composition as described herein; preferably, the kit further comprises an administration device.

### Medicinal use

In yet another aspect, the present invention provides use of the antibody or the antigen binding fragment thereof as described herein, the polynucleotide as described herein, the expression vector as described herein, the host cell as described herein, or the pharmaceutical composition as described herein in the preparation of a medicament for the treatment and/or prevention of a disease or condition mediated by CLDN-18.2; preferably, the disease or condition is cancer.

In yet another aspect, the present invention provides the antibody or the antigen binding fragment thereof as described herein, the polynucleotide as described herein, the expression vector as described herein, the host cell as described herein, or the pharmaceutical composition as described herein for the treatment and/or prevention of a disease or condition mediated by CLDN-18.2, preferably, the disease or condition is cancer.

In yet another aspect, the present invention provides a method of treating and/or preventing a disease or condition mediated by CLDN-18.2, comprising administering to a subject in need thereof the antibody or the antigen binding fragment thereof as described herein, the polynucleotide as described herein, the expression vector as described herein, the host cell as described herein, or the pharmaceutical composition as described herein; preferably, the disease or condition is cancer.

In some embodiments, the cancer is selected from gastric cancer, esophageal cancer, gastroesophageal cancer, pancreatic cancer, cholangiocarcinoma, lung cancer, ovarian cancer, colon cancer, liver cancer, head and neck cancer, gallbladder cancer, intestinal cancer, and bladder cancer.

In some embodiments, modes of administration of the present invention include, but are not limited to, oral, intravenous, subcutaneous, intramuscular, intraarterial, intraarticular (for example, in arthritic joints), via inhalation, aerosol delivery, or intratumoural administration and the like.

In some embodiments, the present invention provides administration in combination with a therapeutically effective amount of one or more therapies (for example, treatment modalities and/or other therapeutic agents) to a subject. In some embodiments, the therapies include surgery and/or radiation therapy.

In some embodiments, the method or use provided in the present invention further comprises administering one or more therapies (for example, treatment modalities and/or other therapeutic agents) to an individual. The antibody of the present invention can be used alone or in combination with other therapeutic agents in the therapy. For example, it can be co-administered with at least one additional therapeutic agent. For example, a PD-1 antibody, a PD-L1 antibody, an LAG-3 antibody and/or a CTLA-4 antibody.

In one embodiment, the method for treating cancer diseases of the present invention further comprises administering an agent for stabilizing or increasing the expression of CLDN-18.2. Expression of CLDN-18.2 is preferably on the cell surface of cancer cells. The agent for stabilizing or increasing the expression of CLDN-18.2 can be oxaliplatin and/or 5-FU.

### Methods for diagnosis and detection

In yet another aspect, the present invention provides a method for detecting the presence of CLDN-18.2 in a sample using the antibody or the antigen binding fragment thereof as described herein. The term "detection" as used herein includes quantitative or qualitative detection. In some embodiments, the sample is a biological sample. In certain embodiments, the biological sample is blood, serum, or other fluid sample of biological origin. In certain embodiments, the biological sample comprises cells or tissues.

The invention includes all combinations of the particular embodiments described. Further embodiments and the full scope of applicability of the present invention will become apparent from the detailed description provided hereinafter. However, it should be understood that the detailed description and specific examples, while indicating preferred embodiments of the present invention, are given by way of illustration only, since various changes and modifications within the spirit and scope of the invention will become apparent to those skilled in the art from this detailed description. All publications, patents, and patent applications cited herein, including quotations, are incorporated herein by reference in their entirety for all purposes.

### Examples

The following examples are provided to demonstrate and further explain some preferred embodiments and aspects of the present invention and should not be construed as limiting the scope thereof.

### Example 1. Preparation of recombinant protein human CLDN-18.2 (hCLDN-18.2) for preparing anti-CLDN-18.2 antibody

The plasmid HG20047-U containing the cDNA sequence of the human CLDN-18 gene was purchased from Sino Biological, Inc., and partial gene fragment of the human CLDN-18 was amplified by PCR using a forward primer 5'-GTACgctagccaccTgaagagcggatccgctcttctTGCCCTGAAATGCATCCGCATTGG CAGC -3' and a reverse primer 5'-GATCgcggccgccctgcaggTTACACATAGTCGTGCTTGGAAGGATAAG -3'. The amplified fragment was digested with NheI and Sbfl, and then cloned into an eukaryotic expression plasmid system (HXP) to obtain a construct HXP-CLDN18P. The obtained construct was sent to Nanjing GenScript Biotechnology Co., Ltd. for synthesis of partial gene sequence of hCLDN-18.2, and the gene sequence was cloned into HXP-CLDN18P by enzyme digestion with BSPQI, to finally obtain a construct HXP-hCLDN-18.2.

### Example 2. Preparation of mouse hybridoma cell

### 2.1. Animal immunization

The HXP-hCLDN-18.2 obtained in Example 1 was mixed with an equal amount of an immune adjuvant (Freund's adjuvant), and five 8-week-old female BALB/c mice were injected intramuscularly for immunization. For the primary immunization, 100 µg of DNA plasmid was used per mouse. Afterwards, booster immunization was carried out every 2 or 3 weeks for a total of 5 times, each time using 50 µg of DNA plasmid per mouse. In the last booster immunization, stable CHO cells overexpressing CLDN-18.2 were used superimposedly, and 1e+7 cells were injected intraperitoneally into each mouse.

### 2.2. Cell fusion

Four days after the last dose of booster immunization, the inguinal lymph node, popliteal lymph node and spleen of the mice were taken, and after grinding in DMEM medium, the suspension rich in lymphocytes was taken, and the lymphocytes were fused with mouse myeloma cells Sp2/0 (ATCC) according to a conventional electrofusion method. The fusion product was cultured in a DMEM complete medium containing 1 : 50 HAT (hypoxanthine, amethopterin and thymidine) for 5 days to screen successfully fused cells (*i*.*e*., hybridoma cells). Then the DMEM complete medium was changed to a DMEM complete medium containing 1 : 50 HT (hypoxanthine and thymidine) until the end of the screening.

The formulation ratio of a DMEM complete medium is: 15% FBS (fetal bovine serum) + 1 : 50 L-glutamine + 100 U/mL of penicillin + 1 : 100 OPI (oxaloacetate, pyruvate and insulin), the incubator condition is 8% CO₂, 37°C.

### Example 3, Screening of mouse hybridoma cells and performance testing of the obtained anti-CLDN-18.2 mouse-derived antibody

Among 3840 different polyclonal hybridoma cell lines, according to the binding reaction at the cell level, the hybridoma cell lines expressing the antibody that can bind to human CLDN-18.2 protein and not bind to the human CLDN18.1 protein were screened out. After the monoclonal hybridoma cell lines were obtained by subcloning, functional screening was carried out according to *in vitro* ADCC and CDC, and finally 7 monoclonal hybridoma cell lines were obtained, corresponding to the expressed antibodies 1H17, 2B19, 4G3, 9J24, 9O24, 10L8 and 10N10 which specifically bind to human CLDN-18.2 recombinant protein and does not bind to human CLDN-18.1 recombinant protein, and have strong ADCC and CDC activities.

### Example 4. Determination of the variable region sequence of the anti-CLDN-18.2 murine antibody represented according to Kabat or IMGT)

The DNA coding sequence corresponding to the variable region of the anti-CLDN-18.2 murine antibody was determined by a method based on degenerate primer PCR. Candidate hybridoma cells were cultured, centrifugated at 1000 rpm and collected, and total RNA was extracted with Trizol. The first-strand cDNA was synthesized using the total RNA as a template, and then the DNA coding sequence corresponding to the variable region was amplified by PCR using the first-strand cDNA as a subsequent template. The primer sequences used in the amplification reaction were complementary to the first framework region of the variable region and the constant region of the antibody (Larrick, J.W. et al., 1990, Scand. J. Immunol., 32, 121-128 and Coloma, J.J. et al., (1991) BioTechniques, 11, 152-156). In a 50 µl reaction system, 1 µl of cDNA, 5 µl of 10 × PCR buffer, 1 µl of each of upstream and downstream primers (25 pmol), 1 µl of dNTP, 1 µl of 25 mmol PL MgCl₂ and 39 µl of H₂O were added and pre-denatured at 95°C for 10 min, then 1 µl of Taq enzyme was added, temperature cycle was performed for PCR amplification. The reaction conditions were 32 cycles of denaturation at 94°C for 1 min, annealing at 58°C for 1 min, and extension at 72°C for 15 s; followed by incubation at 72°C for 10 min. The PCR product was recovered and purified. The amplified product was sequenced to obtain the amino acid sequences of the heavy chain variable region and the light chain variable region of the anti-CLDN-18.2 murine antibody.

The germline and rearranged Ig variable region sequence databases were searched for consensus sequences using NCBI Ig-Blast (http://www.ncbi.nlm.nih.gov/projects/igblast/). Based on Kabat (Wu, T. T and Kabat, E.A. 1970 J. Exp. Med., 132:211-250) and IMGT system (Lefranc M.-P. et al., 1999 Nucleic Acids Research, 27, 209-212), by means of sequence annotation and internet-based sequence analysis (http://www.Imgt.org/IMGT_vquest/share/textes/index.html and http://www.ncbi.nlm.nih.gov/igblast/), the amino acid sequences of the complementarity determine regions (CDRs) were determined.

The amino acid sequences of the light chain and heavy chain variable regions and the CDRs of the anti-CLDN-18.2 murine antibody are shown in the table below:

**Table 1. The amino acid sequences of the CDRs and the variable regions of the anti-CLDN-18.2 murine antibody (KABAT scheme)**

| Murine antibody | 1H17 | 1H17-2 | 2B19 | 4G3 | 9J24 | 9024 | 10L8 | 10N10 |
|---|---|---|---|---|---|---|---|---|
| HCDR1 | SEQ ID NO:1 | SEQ ID NO:1 | SEQ ID NO:10 | SEQ ID NO:16 | SEQ ID NO:22 | SEQ ID NO:28 | SEQ ID NO:34 | SEQ ID NO:37 |
| HCDR2 | SEQ ID NO:2 | SEQ ID NO:2 | SEQ ID NO:11 | SEQ ID NO:17 | SEQ ID NO:23 | SEQ ID NO:29 | SEQ ID NO:35 | SEQ ID NO:38 |
| HCDR3 | SEQ ID NO:3 | SEQ ID NO:3 | SEQ ID NO:12 | SEQ ID NO: 18 | SEQ ID NO:24 | SEQ ID NO:30 | SEQ ID NO:36 | SEQ ID NO:39 |
| LCDR1 | SEQ ID NO:4 | SEQ ID NO:7 | SEQ ID NO: 13 | SEQ ID NO:19 | SEQ ID NO:25 | SEQ ID NO:31 | SEQ ID NO:25 | SEQ ID NO:31 |
| LCDR2 | SEQ ID NO:5 | SEQ ID NO:8 | SEQ ID NO:14 | SEQ ID NO:20 | SEQ ID NO:26 | SEQ ID NO:32 | SEQ ID NO:26 | SEQ ID NO:32 |
| LCDR3 | SEQ ID NO:6 | SEQ ID NO:9 | SEQ ID NO:15 | SEQ ID NO:21 | SEQ ID NO:27 | SEQ ID NO:33 | SEQ ID NO:27 | SEQ ID NO:33 |
| VH | SEQ ID NO:42 | SEQ ID NO:42 | SEQ ID NO:45 | SEQ ID NO:47 | SEQ ID NO:49 | SEQ ID NO:51 | SEQ ID NO:53 | SEQ ID NO:54 |
| VL | SEQ ID NO:43 | SEQ ID NO:44 | SEQ ID NO:46 | SEQ ID NO:48 | SEQ ID NO:50 | SEQ ID NO:52 | SEQ ID NO:50 | SEQ ID NO:52 |

### Example 5. Construction of anti-CLDN-18.2 chimeric antibody

Considering the expression amount, activity, type and the like of the antibody expressed by the hybridoma cells, the anti-CLDN-18.2 murine antibodies 1H17, 1H17-2, 2B19, 4G3, 9J24, and 9O24 were selected to continue the experiment.

The coding sequences of heavy chain constant region Fc and light chain constant region κ were cloned from human blood cells (from Beijing Institute of Blood) and introduced into pCDNA3.1 plasmid. The coding sequences of the heavy chain and light chain variable regions of the aforementioned anti-CLDN-18.2 murine antibody were synthesized by Genscript company. The coding sequences of the heavy chain variable regions and the coding sequences of the light chain variable regions of various anti-CLDN-18.2 murine antibodies were digested with Bspq I, and then introduced by using various combinations as shown in Table 2 into the pCDNA3.1 plasmid into which the coding sequences of the constant regions had been introduced and the correct clones were confirmed through sequencing. Various chimeric heavy chain and light chain expression plasmids were mixed and paired to transfect expression cells, and 5 anti-CLDN-18.2 chimeric antibodies were obtained, with the numbering and the corresponding variable region amino acid sequences shown in Table 2. Subsequent experimental materials were extracted from cells transfected with this series of plasmids.

**Table 2. Numbering of the anti-CLDN-18.2 chimeric antibodies and the sources of their heavy and light chain variable regions**

| Chimeric antibody number | VH | VL |
|---|---|---|
| Chi-JS012-2 | 1H17 VH | 1H17-2 VL |
| Chi-JS012-9 | 2B19 VH | 2B19 VL |
| Chi-JS012-10 | 2B19 VH | 4G3 VL |
| Chi-JS012-21 | 9J24 VH | 2B19 VL |
| Chi-JS012-27 | 9024 VH | 2B19 VL |

### Example 6. Detection of chimeric antibody

### 6.1 Antibody binding affinity:

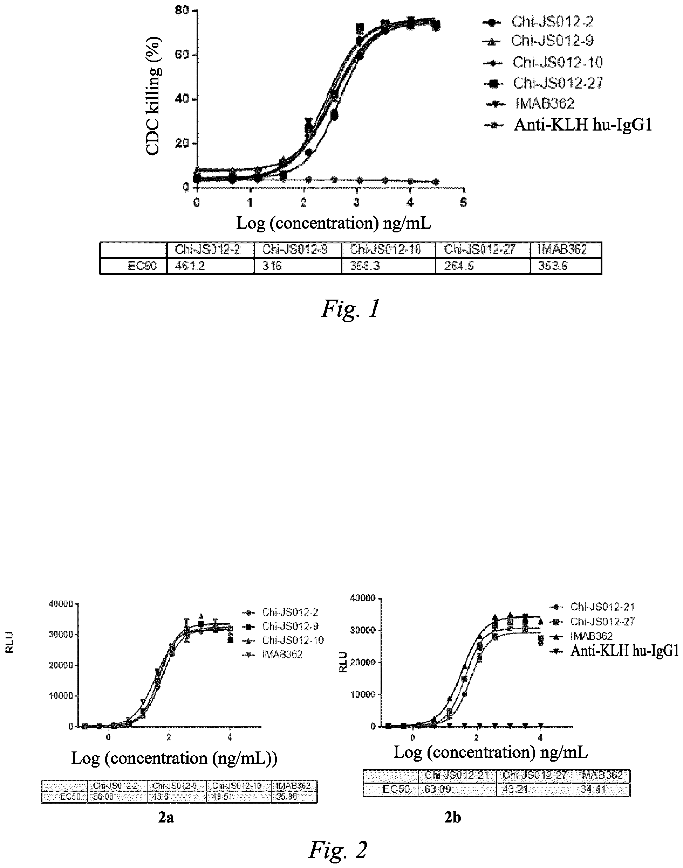

The gastric cancer cell line NUGC4-CLDN-18.2 cells overexpressing human CLDN-18.2 were incubated with the aforementioned anti-CLDN-18.2 chimeric antibodies Chi-JS012-2, Chi-JS012-9, Chi-JS012-10, Chi-JS012-21 and Chi-JS012-27 in a series of gradient dilution concentrations at 4°C for 30 min, respectively, then washed and incubated with a fluorescently labeled secondary antibody. Finally, the fluorescence intensity was detected by BD CantoII flow cytometer. The stronger the fluorescent signal, the higher the affinity of the antibody to the target. The antibody dose-dependent binding curve was fitted by GraphPad (Fig. 1) and EC₅₀ was calculated (Table 3). The positive and negative controls were IMAB362 and anti-KLH hu-IgG1 antibody, respectively.

As shown in Fig. 1 and Table 3, Chi-JS012-2, Chi-JS012-9, Chi-JS012-10, Chi-JS012-21 and Chi-JS012-27 can all bind to human CLDN-18.2 which was highly expressed on that surface of gastric cancer cell line NUGC4-CLDN-18.2.

**Table 3. Cell-level affinity of anti-CLDN-18.2 chimeric antibody (EC₅₀)**

| Chimeric antibody | EC₅₀ (ng/mL) |
|---|---|
| Chi-JS012-2 | 4023 |
| Chi-JS012-9 | 3218 |
| Chi-JS012-10 | 3195 |
| Chi-JS012-21 | 2373 |
| Chi-JS012-27 | 2920 |

### 6.2 Antibody-dependent cell-mediated cytotoxicity (ADCC):

The aforementioned anti-CLDN-18.2 chimeric antibodies Chi-JS012-2, Chi-JS012-9, Chi-JS012-10, Chi-JS012-21 and Chi-JS012-27 in a series of gradient dilution concentrations were incubated with a target cell CHO-CLDN-18.2 overexpressing human CLDN-18.2 (50,000 cells) and an effector cell Jurkat ADCC expressing NFAT-Luc and FcyRIIIaR (100000 cells) at 37°C for 6 h, and the substrate one-glo was then added and the luciferase signal was detected by a microplate reader. Higher fluorescence readings indicated stronger ADCC effects. Antibody dose-dependent ADCC effect curves were fitted by GraphPad (Fig. 2a and 2b), and the positive and negative controls were IMAB362 and anti-KLH hu-IgG1 antibody, respectively.

As shown in Fig. 2a and 2b, the EC₅₀ values for Chi-JS012-2, Chi-JS012-9, Chi-JS012-10, Chi-JS012-21, and Chi-JS012-27 mediated ADCC effects were 56.08 ng/mL, 43.6 ng/mL, 49.51 ng/mL, 63.09 ng/mL, and 43.21 ng/mL, respectively, comparable to the positive control IMAB362 (Fig. 2a: 35.98; Fig. 2b: 034.41 ng/mL).

### 6.3 Complement-dependent cytotoxicity (CDC):

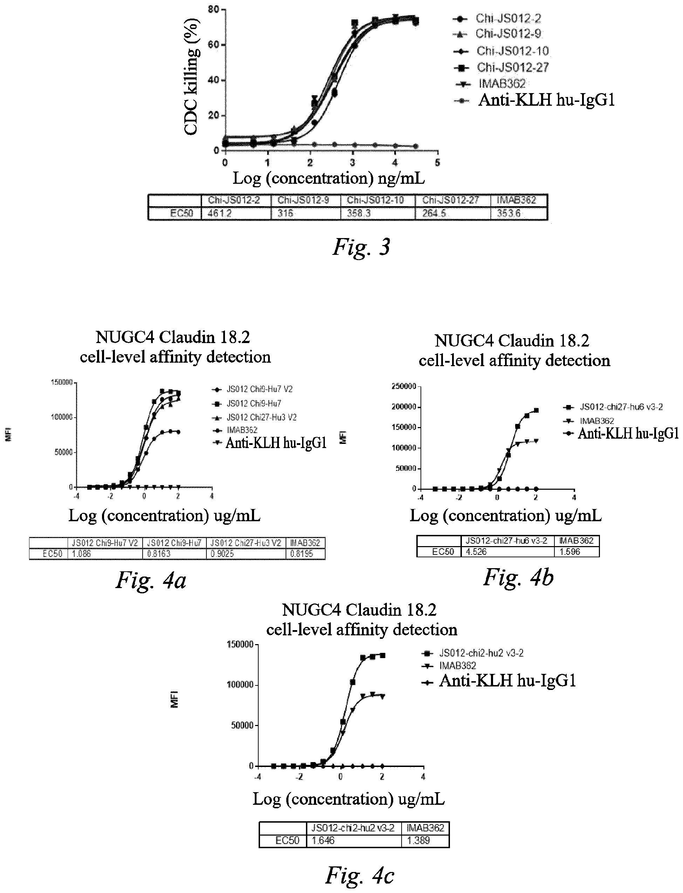

Target cells CHO-CLDN-18.2 overexpressing human CLDN-18.2 (100,000 cells) were mixed with the aforementioned anti-CLDN-18.2 chimeric antibodies Chi-JS012-2, Chi-JS012-9, Chi -JS012-10 and Chi-JS012-27 in a series of gradient dilution concentrations, ten-fold diluted complement serum (Quidel, cat# A113) was added, and incubated at 37°C for 1 h. Finally, the cells were resuspended in PBS and PI dye was added, and after incubation at 4°C for 5 min, the fluorescence intensity was detected with BD Canto II flow cytometer. The antibody dose-dependent complement killing curve was fitted by GraphPad (Fig. 3). The positive and negative controls were IMAB362 and anti-KLH hu-IgG1 antibody, respectively.

As shown in Fig. 3, the EC₅₀ values for Chi-JS012-2, Chi-JS012-9, Chi-JS012-10 and Chi-JS012-27 mediated CDC effects were 461.2 ng/mL, 316 ng/mL, 358.3 ng/mL and 264.5 ng/mL, respectively, comparable to the positive control IMAB362 (353.6 ng/mL).

### Example 7. Humanization of antibody variable regions

For purposes such as reducing the immunogenicity of the antibody, the variable regions of the antibody were humanized and optimized. The optimization results are as follows:
HCDR2: CISSGSGTIYYADTVKG (SEQ ID NO: 2) optimized to **S**ISSGSGTIYYAD**S**VKG (SEQ ID NO: 41) or **Y**ISSGSGTIYYAD**S**VKG (SEQ ID NO: 72);
HCDR2: WINTYTGESTYADDFKG (SEQ ID NO: 11) optimized to WINTYTGESTYAD**G**F**T**G (SEQ ID NO: 40),
HCDR2: TISGGDSYTYYPDSVRG (SEQ ID NO: 29) optimized to TISGGDSYTYYPDSV**K**G (SEQ ID NO: 74);
HCDR3: AYYGNGFSF(SEQ ID NO: 3) optimized to AYYGN**A**FSF (SEQ ID NO: 73);
HCDR3: SYNGNSLPY (SEQ ID NO: 30) optimized to SYN**A**NSLPY (SEQ ID NO: 75);

Thus, a series of humanized anti-CLDN-18.2 antibodies were obtained.

Humanized anti-CLDN-18.2 antibodies JS012-Chi9-hu7, JS012-Chi9-hu7-v2, JS012-Chi27-hu3-v2, JS012-Chi27-hu6-v3-2 and JS012-Chi2-hu2-v3-2 were used in the follow-up experiment. The CDR/variable region sequences of the above antibodies are shown in Tables 4-1 and 4-2, and the amino acid sequences and nucleotide coding sequences of the heavy/light chains are shown in Table 5.

The coding sequences of the heavy chain and light chain variable regions of the humanized anti-CLDN-18.2 antibodies were synthesized by Genscript company, The coding sequences of the heavy chain variable regions and the coding sequences of the light chain variable regions of various synthesized humanized anti-CLDN-18.2 antibodies were digested with Bspq I, and then introduced into the pCDNA3.1 plasmid which comprised the coding sequences of the constant regions and the correct clones were confirmed through sequencing. Various humanized heavy chain and light chain expression plasmids were mixed and paired to transfect expression cells (CHOK1 18, Suzhou Junmeng Biosciences Co., Ltd.), and the expressed antibodies were recovered by centrifugation, and purified according to conventional methods to obtain the humanized anti-CLDN-18.2 antibodies in Tables 4-1 and 4-2.

Among them, the humanized antibody JS012-Chi2-hu2-v3-2 was optimized by adding a glycoform modulator (CDFS01, Shanghai OPM Biosciences Co., Ltd.) during cell culture to obtain the defucosylated antibody JS012-Chi2- hu2-v3-2a. The antibody JS012-Chi2-hu2-v3-2a is a humanized anti-CLDN-18.2 antibody with less than 30% fucosylation.

**Table 4-1. Humanized anti-CLDN-18.2 antibody variable region and CDR sequences (KABAT scheme)**

| Humanized antibody | JS012-Chi9-hu7 | JS012-Chi9-hu7-v2 | JS012-Chi27-hu3-v2 | JS012-Chi27-hu6-v3-2 | JS012-Chi2-hu2-v3-2/JS012-Chi2-hu2-v3-2a |
|---|---|---|---|---|---|
| HCDR1 | SEQ ID NO:10 | SEQ ID NO:10 | SEQ ID NO:28 | SEQ ID NO:28 | SEQ ID NO:1 |
| HCDR2 | SEQ ID NO:40 | SEQ ID NO:40 | SEQ ID NO:29 | SEQ ID NO:29 | SEQ ID NO:41 |
| HCDR3 | SEQ ID NO:12 | SEQ ID NO:12 | SEQ ID NO:30 | SEQ ID NO:30 | SEQ ID NO:2 |
| LCDR1 | SEQ ID NO:13 | SEQ ID NO:13 | SEQ ID NO:13 | SEQ ID NO:13 | SEQ ID NOT |
| LCDR2 | SEQ ID NO:14 | SEQ ID NO:14 | SEQ ID NO:14 | SEQ ID NO:14 | SEQ ID NO:2 |
| LCDR3 | SEQ ID NO:15 | SEQ ID NO:15 | SEQ ID NO:15 | SEQ ID NO:15 | SEQ ID NO:9 |
| VH | SEQ ID NO:55 | SEQ ID NO:57 | SEQ ID NO:58 | SEQ ID NO:59 | SEQ ID NO:60 |
| VL | SEQ ID NO:56 | SEQ ID NO:56 | SEQ ID NO:56 | SEQ ID NO:56 | SEQ ID NO:61 |

**Table 4-2. Humanized anti-CLDN-18.2 antibody variable region and CDR sequences (KABAT scheme)**

| Humanized antibody | JS012-chi2-hu27 | JS012-chi2-hu29 | JS012-chi2-hu10 V2 | JS012-chi9-hu2 | JS012-chi9-hu8 V2 | JS012-chi10-hu9 | JS012-chi27-hu36 | JS012-Chi27-hu3-v2-2 |
|---|---|---|---|---|---|---|---|---|
| HCDR1 | SEQ ID NO:1 | SEQ ID NO:1 | SEQ ID NO:1 | SEQ ID NO:10 | SEQ ID NO:10 | SEQ ID NO:10 | SEQ ID NO:28 | SEQ ID NO:28 |
| HCDR2 | SEQ ID NO:72 | SEQ ID NO:72 | SEQ ID NO:72 | SEQ ID NO:40 | SEQ ID NO:40 | SEQ ID NO:40 | SEQ ID NO:74 | SEQ ID NO:74 |
| HCDR3 | SEQ ID NO:73 | SEQ ID NO:73 | SEQ ID NOT | SEQ ID NO:12 | SEQ ID NO:12 | SEQ ID NO:12 | SEQ ID NO:75 | SEQ ID NO:30 |
| LCDR1 | SEQ ID NO:7 | SEQ ID NO:7 | SEQ ID NO:7 | SEQ ID NO:13 | SEQ ID NO:13 | SEQ ID NO:19 | SEQ ID NO:13 | SEQ ID NO:13 |
| LCDR2 | SEQ ID NO:8 | SEQ ID NO:8 | SEQ ID NO:8 | SEQ ID NO:14 | SEQ ID NO:14 | SEQ ID NO:20 | SEQ ID NO:14 | SEQ ID NO:14 |
| LCDR3 | SEQ ID NO:9 | SEQ ID NO:9 | SEQ ID NO:9 | SEQ ID NO:15 | SEQ ID NO:15 | SEQ ID NO:21 | SEQ ID NO:15 | SEQ ID NO:15 |
| VH | SEQ ID NO:76 | SEQ ID NO:76 | SEQ ID NO:78 | SEQ ID NO:79 | SEQ ID NO:57 | SEQ ID NO:55 | SEQ ID NO:82 | SEQ ID NO:83 |
| VL | SEQ ID NO:61 | SEQ ID NO:77 | SEQ ID NO:61 | SEQ ID NO:80 | SEQ ID NO:56 | SEQ ID NO:81 | SEQ ID NO:56 | SEQ ID NO:56 |
| HC | SEQ ID NO:84 | SEQ ID NO:86 | SEQ ID NO:88 | SEQ ID NO:90 | SEQ ID NO:92 | SEQ ID NO:94 | SEQ ID NO:98 | SEQ ID NO:100 |
| LC | SEQ ID NO:85 | SEQ ID NO:87 | SEQ ID NO:89 | SEQ ID NO:91 | SEQ ID NO:93 | SEQ ID NO:95 | SEQ ID NO:99 | SEQ ID NO:101 |

**Table 5. Amino acid sequences and nucleotide coding sequences of heavy and light chains of humanized anti-CLDN-18.2 antibodies**

| Humanized antibody | Full length sequence | Amino acid sequences | Nucleotide coding sequence |
|---|---|---|---|
| JS012-Chi2-hu2-v3-2 / JS012-Chi2-hu2-v3-2a | HC | SEQ ID NO:64 | SEQ ID NO:66 |
| | LC | SEQ ID NO:65 | SEQ ID NO:67 |
| JS012-Chi9-hu7 | HC | SEQ ID NO:68 | SEQ ID NO:70 |
| | LC | SEQ ID NO:69 | SEQ ID NO:71 |
| JS012-Chi9-hu7-v2 | HC | SEQ ID NO:96 | / |
| | LC | SEQ ID NO:97 | / |

### Example 8. Detection of humanized anti-CLDN-18.2 antibodies

The binding activity of the 5 humanized anti-CLDN-18.2 antibodies JS012-Chi9-hu7, JS012-Chi9-hu7-v2, JS012-Chi27-hu3-v2, JS012-Chi27-hu6-v3-2 and JS012-Chi2-hu2-v3-2 was detected by detecting the binding activity of the antibodies to CLDN-18.2 expressed on the cell surface using FACS. NUGC4-CLDN-18.2 cells were incubated with serially diluted different concentrations of the aforementioned humanized anti-CLDN-18.2 antibodies, then washed and incubated with a fluorescently labeled secondary antibody. Fluorescent signal was detected by FACS; the stronger the detected fluorescent signal, the higher the antibody affinity, that is, the higher the target binding activity. Antibody binding curves were fitted by GraphPad (Fig. 4a, 4b and 4c), and the positive and negative controls were IMAB362 and anti-KLH hu-IgG1 antibody, respectively.

As shown in Fig. 4a, 4b and 4c, JS012-Chi9-hu7-v2, JS012-Chi9-hu7, JS012-Chi27-hu3-v2, JS012-Chi2-hu2-v3-2 and JS012-Chi27-hu6-v3-2 can bind to human CLDN-18.2 highly expressed on the surface of gastric cancer cell line NUGC4 cells, wherein the EC₅₀ of JS012-Chi9-hu7-v2, JS012-Chi9-hu7, JS012-Chi27-hu3-v2 and JS012-Chi2-hu2-v3-2 were comparable to the positive control.

### Example 9. ADCC of humanized anti-CLDN-18.2 antibodies

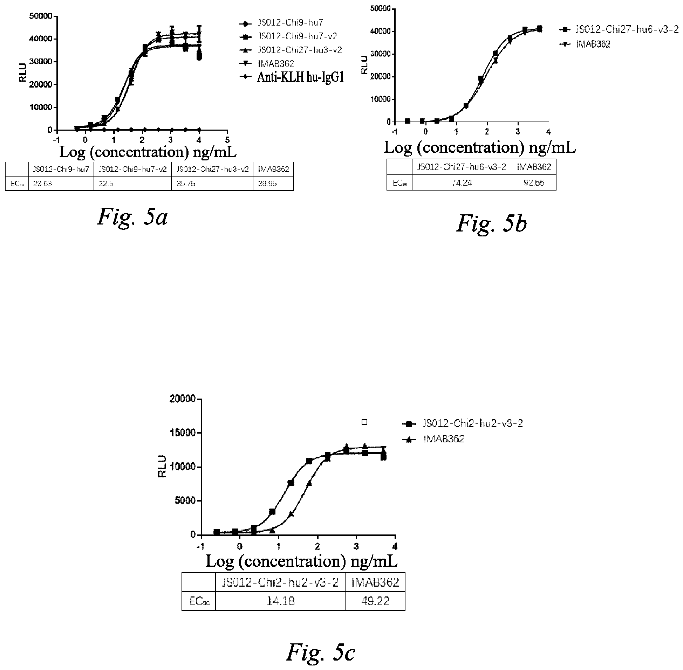

The humanized anti-CLDN-18.2 antibodies were of human IgG1 subtype and can mediate the ADCC effect. The Fc end of the antibodies binds to the FcyIIIaR receptor on the surface of NK cells and activates NK cells to kill target cells. Using the NFAT pathway luciferase system expressing FcyIIIaR, the *in vivo* ADCC effect was simulated by a reporter gene system. The humanized anti-CLDN-18.2 antibodies were co-incubated with the target cells CHO-CLDN-18.2 cells and the Jurkat ADCC effector cells, and the signal was detected with a microplate reader after adding the substrate one-glo. The data were analyzed with GraphPad to compare the dose-dependent ADCC effects of the antibodies (Fig. 5a, 5b, and 5c). The positive and negative controls were IMAB362 and anti-KLH hu-IgG1 antibody, respectively.

As shown in Fig. 5a, 5b and 5c, the EC₅₀ values for the JS012-Chi9-hu7-v2, JS012-Chi9-hu7, JS012-Chi27-hu3-v2, JS012-Chi2-hu2-v3-2 and JS012-Chi27-hu6-v3-2 mediated ADCC effects were 22.5 ng/mL, 23.63 ng/mL, 35.75 ng/mL, 14.18 ng/mL, and 74.24 ng/mL, respectively, all significantly better than the positive control IMAB362.

### Example 10. CDC of humanized anti-CLDN-18.2 antibodies

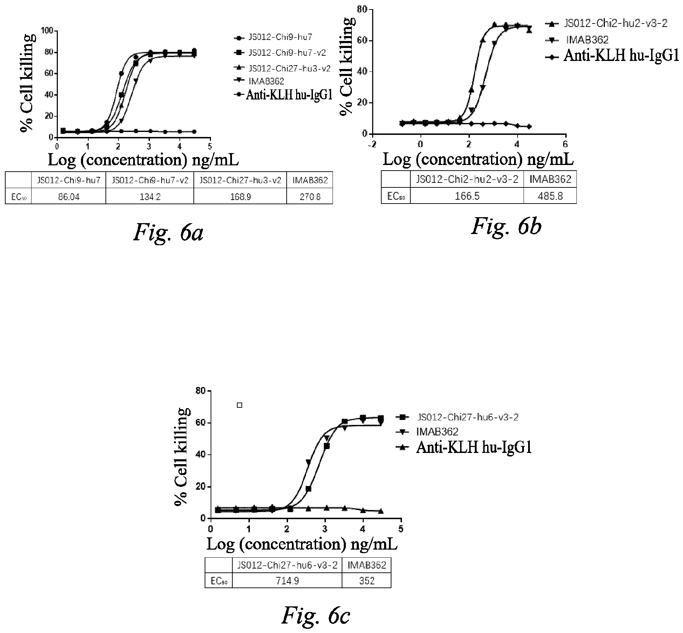

The humanized anti-CLDN-18.2 antibodies can also mediate the CDC effect and kill target cells by forming membrane attack complexes. Complement serum (diluted 1 : 10) was incubated with the humanized anti-CLDN-18.2 antibodies and target cells CHO-CLDN-18.2 (100,000 cells) in a 37°C incubator for 1 hour, and the survival and killing of target cells were detected by propidium iodide (PI) staining to calculate the relative killing rate. The dose-dependent CDC effect of the humanized anti-CLDN-18.2 antibodies was reflected by the relative killing rate (Fig. 6a, 6b and 6c). The positive and negative controls were IMAB362 and anti-KLH hu-IgG1 antibody, respectively.

As shown in Fig. 6a, 6b and 6c, the EC₅₀ values for the JS012-Chi9-hu7-v2, JS012-Chi9-hu7, JS012-Chi27-hu3-v2, JS012-Chi2-hu2-v3-2 and JS012-Chi27-hu6-v3-2 mediated CDC effects were 134.2 ng/mL, 86.04 ng/mL, 168.9 ng/mL, 166.5 ng/mL and 714.9 ng/mL, respectively, wherein JS012-Chi9-hu7-v2, JS012-Chi9-hu7, JS012-Chi27-hu3-v2 and JS012-Chi2-hu2-v3-2 were significantly better than the positive control IMAB362.

### Example 11: Inhibitory effect of humanized anti-CLDN-18.2 antibodies on tumour growth of human gastric cancer MKN45 hClaudin18.2 Mixeno transplanted in M-NSG mice

### 1. Testing purpose

Evaluating the anti-tumour effect of JS012-Chi2-hu2 v3-2a of the present invention in the subcutaneous transplantation model of human gastric cancer MKN45 hClaudin18.2 Mixeno.

### 2. Testing process

5 × 10⁶ MKN45 hClaudin18.2 tumour cells (Shanghai Novobiosci Co., Ltd) were mixed with 5 × 10⁶ PBMC cells (Shanghai OriBiotech Co., Ltd), inoculated subcutaneously on the right back of M-NSG mice (Shanghai Model Organisms Center, Inc) at 0.2 ml/mouse (culture medium RPMI1640 (Gibco) containing cells and 50% Matrigel) and according to body weight, 50 mice were selected and randomly divided into the following 5 groups (10 mice/group)
Anti-KLH hIgG1 negative control group, 10 mg/kg;
JS012-Chi2-hu2 v3-2a treatment group, 1 mg/kg;
JS012-Chi2-hu2 v3-2a treatment group, 3 mg/kg;
JS012-Chi2-hu2 v3-2a treatment group, 10 mg/kg;
and IMAB362 positive control group, 10 mg/kg.

On the day of grouping, administration was performed 4 hours after the tumour cells were inoculated. The route of administration for all groups was intraperitoneal injection. The administration was performed twice a week for 9 consecutive administrations. The experiment ended 3 days after the last administration. The tumour volume and body weight were measured twice a week, and the body weight and tumour volume of the mice were recorded. At the end of the experiment, the mice were euthanized, and the tumour inhibition rate TGI% was calculated (TGI % = [1-T/C] × 100%). (T: the mean tumour volume of the treatment group or the positive control group at the end of the experiment, C: the mean tumour volume of the negative control group at the end of the experiment).

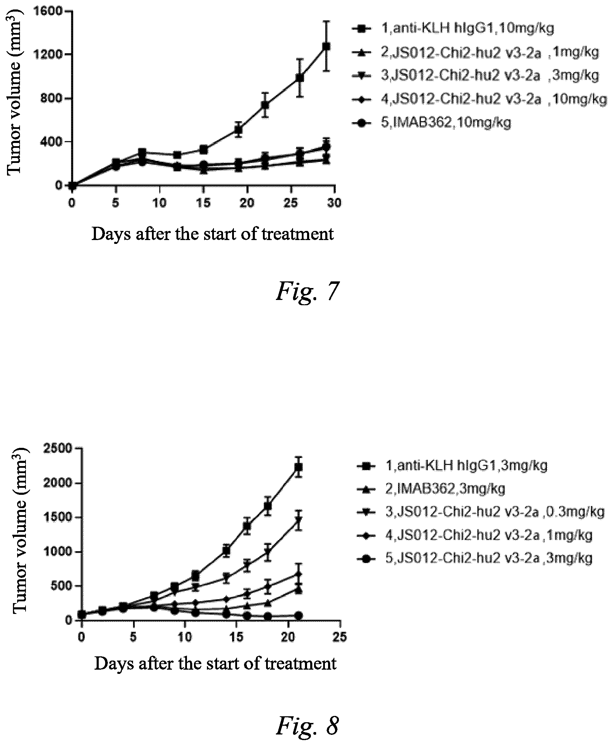

As shown in Fig. 7, at the end of the experiment, the mean tumour volume of the anti-KLH hIgG1 negative control group was 1276 ± 228 mm³. The mean tumour volumes of JS012-Chi2-hu2a v3-2 at 1, 3, and 10 mg/kg doses were 235 ± 25 mm³, 243 ± 33 mm³, and 343 ± 63 mm³, respectively, and the TGI were 81.6%, 81.0%, and 73.1%, respectively, showing significant tumour inhibition effect. The mean tumour volume of IMAB362 at 10 mg/kg dose was 361 ± 73 mm³ and the TGI was 71.7%. Under the condition of the same dose (10 mg/kg), the tumour inhibition effect of JS012-Chi2-hu2 v3-2a was slightly better than that of IMAB362.

### Example 12: Inhibitory effect of humanized anti-CLDN-18.2 antibodies on tumour growth of human pancreatic cancer hCLDN18.2 MIA PaCa-2 transplanted in CB-17 SCID mice

### 1. Testing purpose

To evaluate the anti-tumour effect of JS012-Chi2-hu2 v3-2a of the present invention in the subcutaneous transplantation model of human pancreatic cancer hCLDN18.2 MIA PaCa-2.

### 2. Testing process

5 × 10⁶ hCLDN18.2 MIA PaCa-2 cells (Accurusbio-C3002) were inoculated subcutaneously on the right back of CB-17 SCID mice (Shanghai JiHui Experimental Animal Co., Ltd.) at 0.2 ml/mouse (culture medium DMEM (Gibco) containing cells and 50% Matrigel), and when the mean tumour volume was about 89 mm³, according to the tumour volume, 40 mice were selected and randomly divided into the following 5 groups (8 mice/group):
Anti-KLH hIgG1 negative control group, 3 mg/kg;
IMAB362 positive control group, 3 mg/kg;
JS012-Chi2-hu2 v3-2a treatment group, 0.3 mg/kg;
JS012-Chi2-hu2 v3-2a treatment group, 1 mg/kg;
and JS012-Chi2-hu2 v3-2a treatment group, 3 mg/kg.

On the day of grouping, administration was performed. The route of administration for all groups was intraperitoneal injection. The administration was performed twice a week for 6 consecutive administrations. The experiment ended 4 days after the last administration. The tumour volume and body weight were measured 3 times a week, and the body weight and tumour volume of the mice were recorded. At the end of the experiment, the mice were euthanized, and the tumour inhibition rate TGI% was calculated (TGI % = [1-(Ti-T0)/(Vi-V0)] × 100%). (Ti: the mean tumour volume of the treatment group or the positive control group at day i post-administration, T0: the mean tumour volume of the treatment group or the positive control group on day 0 post-administration; Vi: the mean tumour volume of the negative control group on day i post-administration, and V0: the mean tumour volume of the negative control group on day 0 of post-administration).

As shown in Fig. 8, at the end of the experiment, the mean tumour volume of the anti-KLH hIgG1 group was 2235 ± 145 mm3. The mean tumour volume of IMAB362 at 3 mg/kg dose was 465 ± 74 mm3 and the TGI was 82.5%. The mean tumour volumes of JS012-Chi2-hu2 v3-2a at 0.3, 1 and 3 mg/kg doses were 1455 ± 142 mm3, 673 ± 153 mm3 and 74 ± 19 mm3, respectively, and the TGI were 36.3%, 72.8% and 100.6%, respectively, showing significant tumour inhibition effect. Under the condition of the same dose (3 mg/kg), the tumour inhibition effect of JS012-Chi2-hu2 v3-2a was significantly better than that of IMAB362.

Sequence Listing

SEQ ID NO: 1
NFGMH

SEQ ID NO: 2
CISSGSGTIYYADTVKG

SEQ ID NO: 3
AYYGNGFSF

SEQ ID NO: 4
SASSIFSYIF

SEQ ID NO: 5
LTSKLAS

SEQ ID NO: 6
HQWKITHT

SEQ ID NO: 7
KSSQSLLNSGNQKNYLT

SEQ ID NO: 8
WASTRES

SEQ ID NO: 9
QNVYSYPLT

SEQ ID NO: 10
NYGMN

SEQ ID NO: 11
WINTYTGESTYADDFKG

SEQ ID NO: 12
TLGYGNSLAY

SEQ ID NO: 13
KSSQSLLNSGNQRNYLT

SEQ ID NO: 14
WASTRES

SEQ ID NO: 15
QNNYFYPFT

SEQ ID NO: 16
SFGVS

SEQ ID NO: 17
AIWAGGNRNYNSALMS

SEQ ID NO: 18
GSYGSSFAN

SEQ ID NO: 19
KSSQSLLNSGNQKNYLT

SEQ ID NO: 20
WASTRDS

SEQ ID NO: 21
QNAYFYPFT

SEQ ID NO: 22
NYGMN

SEQ ID NO: 23
WINTYTGESTYADDFKG

SEQ ID NO: 24
SYNGNSLPY

SEQ ID NO: 25

KSSQSLLNSGNQKNYLT

SEQ ID NO: 26
WASTRES

SEQ ID NO: 27
QNDYSYPLT

SEQ ID NO: 28
SYGMS

SEQ ID NO: 29
TISGGDSYTYYPDSVRG

SEQ ID NO: 30
SYNGNSLPY

SEQ ID NO: 31
KSSQSLLNSGNQKNYLT

SEQ ID NO: 32
WASTRES

SEQ ID NO: 33
QNTYSFPLT

SEQ ID NO: 34
NYGMN

SEQ ID NO: 35
WINTYTGEPTYADDFKG

SEQ ID NO: 36
GY

SEQ ID NO: 37
NYLIE

SEQ ID NO: 38
VINPGSGGTNYNEKFKG

SEQ ID NO: 39
PHDGYYFWFAY

SEQ ID NO: 40
WINTYTGESTYADGFTG

SEQ ID NO: 41
SISSGSGTIYYADSVKG

SEQ ID NO: 42
DVQLVESGGGLVQPGGSRKLSCAASGFTLNNFGMHWVRQAPEKGLEWVACISSGSGTIYYA
DTVKGRFTISRDNPKNTLFLQMTSLRSEDTAMYYCARAYYGNGFSFWGQGTLVTVSS

SEQ ID NO: 43
QIVLTQSPTLMSASPGEKVTMTCSASSIFSYIFWSQQKPRSSPKPWIYLTSKLASGVPVRF
SGSGSGTSYSLTISTMEAEDAATYYCHQWKITHTFGAGTKLELK

SEQ ID NO: 44
DIVMTQSPSSLTVTAGEKVTMSCKSSQSLLNSGNQKNYLTWYQQKPGQPPKLLIYWASTRE
SGVPDRFTGSGSGTDFTLTISSVQAEDLAIYYCQNVYSYPLTFGAGTKLEIK

SEQ ID NO: 45
QIQLVQSGPELKKPGETVKISCKASGYTFTNYGMNWVKQAPGKGLKWMGWINTYTGESTYA
DDFKGRFAFSSETSARTAYLQINNLKNEDMATYFCATLGYGNSLAYWGQGTLVTVSS

SEQ ID NO: 46
DIVMTQYPSFLTVTTGEKVTMSCKSSQSLLNSGNQRNYLTWYQQKPGQPPKLLIYWASTRE
SGVPDRFAGSGSGTDFTLTISSVQAEDLAIYYCQNNYFYPFTFGSGTKLEVK

SEQ ID NO: 47
QVQLKESGPGLVAPSQSLSITCTVSGFSLTSFGVSWVRQPPGKGLEWLGAIWAGGNRNYNS
ALMSRLSISKDNSKSQVFLKMTSLQTDDTAIYYCARGSYGSSFANWGQGTLVTVSS

SEQ ID NO: 48

DIVMTQSPSFLTVTAGEKVTMSCKSSQSLLNSGNQKNYLTWYQQKPGQPPKLLIYWASTRD
SGVPDRFTGSGSGTDFTLTITSVQAEDLAVFYCQNAYFYPFTFGSGTKLEIK

SEQ ID NO: 49
QIQLVQSGPELKKPGETVKISCKASGYTFTNYGMNWVKQAPGKGLKWMGWINTYTGESTYA
DDFKGRFAFSSETSARTAYLQINNLKNEGMATYFCARSYNGNSLPYWGQGTLVTVSS

SEQ ID NO: 50
DIVMTQSPSFLTVTAGEKVTMSCKSSQSLLNSGNQKNYLTWYQQKPGQPPKLLIYWASTRE
SGVPDRFTGSGSGTDFTLTISSVQAEDLAVYYCQNDYSYPLTFGAGTKLELK

SEQ ID NO: 51
EVKLVESGGGLVKPGGSLKLSCAASGFTFSSYGMSWVRQTPEKRLECVATISGGDSYTYYP
DSVRGRFTISRDIAKNNLYLQMSSLRSEDTALYYCARSYNGNSLPYWGQGTLVTVSS

SEQ ID NO: 52
DIVMTQSPSSLTVTAGEKVTMSCKSSQSLLNSGNQKNYLTWYQQKPGQPPKLLIYWASTRE
SGVPDRFTGSGSGIDFTLTISSVQAEDLAFYYCQNTYSFPLTFGAGTRLDLK

SEQ ID NO: 53
QIQLVQSGPELKKPGETVKISCKASGYTFTNYGMNWVKQAPGKGLKWMGWINTYTGEPTYA
DDFKGRFAFSLETSASTAYLQINNLKNEDTATYFCARGYWGQGTTLTVSS

SEQ ID NO: 54
QVQLQQSGAELVRPGTSVKVSCKASGYAFTNYLIEWVKQRPGQGLEWIGVINPGSGGTNYN
EKFKGKATLTADKSSSTAYMQLSSLTSDDSAVYFCARPHDGYYFWFAYWGQGTLVTVSS

SEQ ID NO: 55
QIQLVQSGSELKKPGESVKVSCKASGYTFTNYGMNWVRQAPGQGLEWMGWINTYTGESTYA
DGFTGRFVFSSDTSARTAYLQISSLKAEDTAVYFCATLGYGNSLAYWGQGTLVTVSS

SEQ ID NO: 56
DIVMTQSPDSLAVSLGERATINCKSSQSLLNSGNQRNYLTWYQQKPGQPPKLLIYWASTRE
SGVPDRFSGSGSGTDFTLTISSLQAEDVAVYYCQNNYFYPFTFGQGTKLEIK

SEQ ID NO: 57
QVQLVQSGSELKKPGESVKVSCKASGYTFTNYGMNWVRQAPGQGLEWMGWINTYTGESTYA
DGFTGRFVFSMDTSASTAYLQISSLKAEDMAMYFCATLGYGNSLAYWGQGTLVTVSS

SEQ ID NO: 58
EVQLVESGGGLVKPGGSLRLSCAASGFTFSSYGMSWVRQAPGKGLEWVSTISGGDSYTYYP
DSVRGRFTISRDNAKNSLYLQMNSLRAEDTAVYYCARSYNGNSLPYWGQGTLVTVSS

SEQ ID NO: 59
EVQLVESGGGLVKPGGSLRLSCAASGFTFSSYGMSWVRQAPGKGLESVSTISGGDSYTYYP
DSVRGRFTISRDIAKNSLYLQMNSLRAEDTAVYYCARSYNGNSLPYWGQGTLVTVSS

SEQ ID NO: 60
EVQLVESGGGLVQPGGSLRLSCAASGFTLNNFGMHWVRQAPGKGLEWVSSISSGSGTIYYA
DSVKGRFTISRDNPKNSLYLQMNSLRAEDTAVYYCARAYYGNGFSFWGQGTLVTVSS

SEQ ID NO: 61
DIVMTQSPDSLAVSLGERATINCKSSQSLLNSGNQKNYLTWYQQKPGQPPKLLIYWASTRE
SGVPDRFSGSGSGTDFTLTISSLQAEDVAVYYCQNVYSYPLTFGQGTKLEIK

SEQ ID NO: 62 X1ISSGSGTIYYADX2VKG

SEQ ID NO: 63 WINTYTGESTYADX3FX4G

SEQ ID NO: 64
EVQLVESGGGLVQPGGSLRLSCAASGFTLNNFGMHWVRQAPGKGLEWVSSISSGSGTIYYA
DSVKGRFTISRDNPKNSLYLQMNSLRAEDTAVYYCARAYYGNGFSFWGQGTLVTVSSASTK
GPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCS
VMHEALHNHYTQKSLSLSPGK

SEQ ID NO: 65
DIVMTQSPDSLAVSLGERATINCKSSQSLLNSGNQKNYLTWYQQKPGQPPKLLIYWASTRE
SGVPDRFSGSGSGTDFTLTISSLQAEDVAVYYCQNVYSYPLTFGQGTKLEIKRTVAAPSVF
IFPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC

SEQ ID NO: 66

GAGGTGCAGCTGGTGGAGAGCGGCGGCGGCCTGGTGCAGCCCGGCGGCAGCCTGAGACTGA
GCTGCGCCGCCAGCGGCTTCACCCTGAACAACTTCGGCATGCACTGGGTGAGACAGGCCCC
CGGCAAGGGCCTGGAGTGGGTGAGCTcCATCAGCAGCGGCAGCGGCACCATCTACTACGCC
GACAGCGTGAAGGGCAGATTCACCATCAGCAGAGACAACCCCAAGAACAGCCTGTACCTGC
AGATGAACAGCCTGAGAGCCGAGGACACCGCCGTGTACTACTGCGCCAGAGCCTACTACGG
CAACGGCTTCAGCTTCTGGGGCCAGGGCACCCTGGTGACCGTGAGCAGCGCTAGCACCAAG
GGCCCATCCGTCTTCCCCCTGGCACCCTCCTCCAAGAGCACCTCTGGGGGCACAGCGGCCC
TGGGCTGCCTGGTCAAGGACTACTTCCCCGAACCGGTGACGGTGTCGTGGAACTCAGGCGC
CCTGACCAGCGGCGTGCACACCTTCCCGGCTGTCCTACAGTCCTCAGGACTCTACTCCCTC
AGCAGCGTGGTGACCGTGCCCTCCAGCAGCTTGGGCACCCAGACCTACATCTGCAACGTGA
ATCACAAGCCCAGCAACACCAAGGTGGACAAGAAAGTTGAGCCCAAATCTTGTGACAAAAC
TCACACATGCCCACCGTGCCCAGCACCTGAACTCCTGGGGGGACCGTCAGTCTTCCTCTTC
CCCCCAAAACCCAAGGACACCCTCATGATCTCCCGGACCCCTGAGGTCACATGCGTGGTGG
TGGACGTGAGCCACGAAGACCCTGAGGTCAAGTTCAACTGGTACGTGGACGGCGTGGAGGT
GCATAATGCCAAGACAAAGCCGCGGGAGGAGCAGTACAACAGCACGTACCGTGTGGTCAGC
GTCCTCACCGTCCTGCACCAGGACTGGCTGAATGGCAAGGAGTACAAGTGCAAGGTCTCCA
ACAAAGCCCTCCCAGCCCCCATCGAGAAAACCATCTCCAAAGCCAAAGGGCAGCCCCGAGA
ACCACAGGTGTACACCCTGCCCCCATCCCGGGACGAGCTGACCAAGAACCAGGTCAGCCTG
ACCTGCCTGGTCAAAGGCTTCTATCCCAGCGACATCGCCGTGGAGTGGGAGAGCAATGGGC
AGCCGGAGAACAACTACAAGACCACGCCTCCCGTGCTGGACTCCGACGGCTCCTTCTTCCT
CTATAGCAAGCTCACCGTGGACAAGAGCAGGTGGCAGCAGGGGAACGTCTTCTCATGCTCC
GTGATGCATGAGGCTCTGCACAACCACTACACGCAGAAGTCCCTCTCCCTGTCTCCGGGTA
AATGA

SEQ ID NO: 67
GACATCGTGATGACCCAGAGCCCCGACAGCCTGGCCGTGAGCCTGGGCGAGAGAGCCACCA
TCAACTGCAAGAGCAGCCAGAGCCTGCTGAACAGCGGCAACCAGAAGAACTACCTGACCTG
GTACCAGCAGAAGCCCGGCCAGCCCCCCAAGCTGCTGATCTACTGGGCCAGCACCAGAGAG
AGCGGCGTGCCCGACAGATTCAGCGGCAGCGGCAGCGGCACCGACTTCACCCTGACCATCA
GCAGCCTGCAGGCCGAGGACGTGGCCGTGTACTACTGCCAGAACGTGTACAGCTACCCCCT
GACCTTCGGCCAGGGCACCAAGCTGGAGATCAAGCGAACTGTGGCTGCACCATCTGTCTTC
ATCTTCCCGCCATCTGATGAGCAGTTGAAATCTGGAACTGCCTCTGTTGTGTGCCTGCTGA
ATAACTTCTATCCCAGAGAGGCCAAAGTACAGTGGAAGGTGGATAACGCCCTCCAATCGGG
TAACTCCCAGGAGAGTGTCACAGAGCAGGACAGCAAGGACAGCACCTACAGCCTCAGCAGC
ACCCTGACGCTGAGCAAAGCAGACTACGAGAAACACAAAGTCTACGCCTGCGAAGTCACCC
ATCAGGGCCTGAGCTCGCCCGTCACAAAGAGCTTCAACAGGGGAGAGTGTTAG

SEQ ID NO: 68

QIQLVQSGSELKKPGESVKVSCKASGYTFTNYGMNWVRQAPGQGLEWMGWINTYTGESTYA
DGFTGRFVFSSDTSARTAYLQISSLKAEDTAVYFCATLGYGNSLAYWGQGTLVTVSSASTK
GPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPSVFLF
PPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSRDELTKNQVSL
TCLVKGFYPSDIAVEWESNGQPENNYKTTPPVLDSDGSFFLYSKLTVDKSRWQQGNVFSCS
VMHEALHNHYTQKSLSLSPGK

SEQ ID NO: 69
DIVMTQSPDSLAVSLGERATINCKSSQSLLNSGNQRNYLTWYQQKPGQPPKLLIYWASTRE
SGVPDRFSGSGSGTDFTLTISSLQAEDVAVYYCQNNYFYPFTFGQGTKLEIKRTVAAPSVF
IFPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC

SEQ ID NO: 70
CAGATCCAGCTGGTGCAGAGCGGCAGCGAGCTGAAGAAGCCCGGCGAGAGCGTGAAGGTGA
GCTGCAAGGCCAGCGGCTACACCTTCACCAACTACGGCATGAACTGGGTGAGACAGGCCCC
CGGCCAGGGCCTGGAGTGGATGGGCTGGATCAACACCTACACCGGCGAGAGCACCTACGCC
GACGGCTTCACCGGCAGATTCGTGTTCAGCAGCGACACCAGCGCCAGAACCGCCTACCTGC
AGATCAGCAGCCTGAAGGCCGAGGACACCGCCGTGTACTTCTGCGCCACCCTGGGCTACGG
CAACAGCCTGGCCTACTGGGGCCAGGGCACCCTGGTGACCGTGAGCAGCGCTAGCACCAAG
GGCCCATCCGTCTTCCCCCTGGCACCCTCCTCCAAGAGCACCTCTGGGGGCACAGCGGCCC
TGGGCTGCCTGGTCAAGGACTACTTCCCCGAACCGGTGACGGTGTCGTGGAACTCAGGCGC
CCTGACCAGCGGCGTGCACACCTTCCCGGCTGTCCTACAGTCCTCAGGACTCTACTCCCTC
AGCAGCGTGGTGACCGTGCCCTCCAGCAGCTTGGGCACCCAGACCTACATCTGCAACGTGA
ATCACAAGCCCAGCAACACCAAGGTGGACAAGAAAGTTGAGCCCAAATCTTGTGACAAAAC
TCACACATGCCCACCGTGCCCAGCACCTGAACTCCTGGGGGGACCGTCAGTCTTCCTCTTC
CCCCCAAAACCCAAGGACACCCTCATGATCTCCCGGACCCCTGAGGTCACATGCGTGGTGG
TGGACGTGAGCCACGAAGACCCTGAGGTCAAGTTCAACTGGTACGTGGACGGCGTGGAGGT
GCATAATGCCAAGACAAAGCCGCGGGAGGAGCAGTACAACAGCACGTACCGTGTGGTCAGC
GTCCTCACCGTCCTGCACCAGGACTGGCTGAATGGCAAGGAGTACAAGTGCAAGGTCTCCA
ACAAAGCCCTCCCAGCCCCCATCGAGAAAACCATCTCCAAAGCCAAAGGGCAGCCCCGAGA
ACCACAGGTGTACACCCTGCCCCCATCCCGGGACGAGCTGACCAAGAACCAGGTCAGCCTG
ACCTGCCTGGTCAAAGGCTTCTATCCCAGCGACATCGCCGTGGAGTGGGAGAGCAATGGGC
AGCCGGAGAACAACTACAAGACCACGCCTCCCGTGCTGGACTCCGACGGCTCCTTCTTCCT
CTATAGCAAGCTCACCGTGGACAAGAGCAGGTGGCAGCAGGGGAACGTCTTCTCATGCTCC

GTGATGCATGAGGCTCTGCACAACCACTACACGCAGAAGTCCCTCTCCCTGTCTCCGGGTA
AATGA

SEQ ID NO: 71
GACATCGTGATGACCCAGAGCCCCGACAGCCTGGCCGTGAGCCTGGGCGAGAGAGCCACCA
TCAACTGCAAGAGCAGCCAGAGCCTGCTGAACAGCGGCAACCAGAGAAACTACCTGACCTG
GTACCAGCAGAAGCCCGGCCAGCCCCCCAAGCTGCTGATCTACTGGGCCAGCACCAGAGAG
AGCGGCGTGCCCGACAGATTCAGCGGCAGCGGCAGCGGCACCGACTTCACCCTGACCATCA
GCAGCCTGCAGGCCGAGGACGTGGCCGTGTACTACTGCCAGAACAACTACTTCTACCCCTT
CACCTTCGGCCAGGGCACCAAGCTGGAGATCAAGCGAACTGTGGCTGCACCATCTGTCTTC
ATCTTCCCGCCATCTGATGAGCAGTTGAAATCTGGAACTGCCTCTGTTGTGTGCCTGCTGA
ATAACTTCTATCCCAGAGAGGCCAAAGTACAGTGGAAGGTGGATAACGCCCTCCAATCGGG
TAACTCCCAGGAGAGTGTCACAGAGCAGGACAGCAAGGACAGCACCTACAGCCTCAGCAGC
ACCCTGACGCTGAGCAAAGCAGACTACGAGAAACACAAAGTCTACGCCTGCGAAGTCACCC
ATCAGGGCCTGAGCTCGCCCGTCACAAAGAGCTTCAACAGGGGAGAGTGTTAG

SEQ ID NO: 72
YISSGSGTIYYADSVKG

SEQ ID NO: 73
AYYGNAFSF

SEQ ID NO: 74
TISGGDSYTYYPDSVKG

SEQ ID NO: 75
SYNANSLPY

SEQ ID NO: 76
EVQLVESGGGLVQPGGSLRLSCAASGFTLNNFGMHWVRQAPGKGLEWVSYISSGSGTIYYA
DSVKGRFTISRDNPKNSLYLQMNSLRAEDTAVYYCARAYYGNAFSFWGQGTLVTVSS

SEQ ID NO: 77
DIVMTQSPSSLAVSLGERATINCKSSQSLLNSGNQKNYLTWYQQKPGQPPKLLIYWASTRE
SGVPDRFSGSGSGTDFTLTISSLQAEDVAVYYCQNVYSYPLTFGQGTKLEIK

SEQ ID NO: 78

EVQLVESGGGLVQPGGSLRLSCAASGFTLNNFGMHWVRQAPGKGLEWVSYISSGSGTIYYA
DSVKGRFTISRDNPKNSLYLQMNSLRAEDTAVYYCARAYYGNGFSFWGQGTLVTVSS

SEQ ID NO: 79
QIQLVQSGSELKKPGASVKVSCKASGYTFTNYGMNWVRQAPGQGLEWMGWINTYTGESTYA
DGFTGRFVFSSDTSARTAYLQISSLKAEDTAVYFCATLGYGNSLAYWGQGTLVTVSS

SEQ ID NO: 80
DIVMTQSPDSLAVTTGERATINCKSSQSLLNSGNQRNYLTWYQQKPGQPPKLLIYWASTRE
SGVPDRFSGSGSGTDFTLTISSLQAEDVAVYYCQNNYFYPFTFGQGTKLEIK

SEQ ID NO: 81
DIVMTQSPDFLAVSLGERATINCKSSQSLLNSGNQKNYLTWYQQKPGQPPKLLIYWASTRD
SGVPDRFSGSGSGTDFTLTISSLQAEDVAVYYCQNAYFYPFTFGPGTKVDIK

SEQ ID NO: 82
EVQLVESGGGLVKPGGSLRLSCAASGFTFSSYGMSWVRQAPGKGLEWVSTISGGDSYTYYP
DSVKGRFTISRDIAKNSLYLQMNSLRAEDTAVYYCARSYNANSLPYWGQGTLVTVSS

SEQ ID NO: 83
EVQLVESGGGLVKPGGSLRLSCAASGFTFSSYGMSWVRQAPGKGLEWVSTISGGDSYTYYP
DSVKGRFTISRDNAKNSLYLQMNSLRAEDTAVYYCARSYNGNSLPYWGQGTLVTVSS

SEQ ID NO: 84
EVQLVESGGGLVQPGGSLRLSCAASGFTLNNFGMHWVRQAPGKGLEWVSYISSGSGTIYYADSV
KGRFTISRDNPKNSLYLQMNSLRAEDTAVYYCARAYYGNAFSFWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK
SEQ ID NO: 85
DIVMTQSPDSLAVSLGERATINCKSSQSLLNSGNQKNYLTWYQQKPGQPPKLLIYWASTRESGV
PDRFSGSGSGTDFTLTISSLQAEDVAVYYCQNVYSYPLTFGQGTKLEIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 86

EVQLVESGGGLVQPGGSLRLSCAASGFTLNNFGMHWVRQAPGKGLEWVSYISSGSGTIYYADSV
KGRFTISRDNPKNSLYLQMNSLRAEDTAVYYCARAYYGNAFSFWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 87
DIVMTQSPSSLAVSLGERATINCKSSQSLLNSGNQKNYLTWYQQKPGQPPKLLIYWASTRESGV
PDRFSGSGSGTDFTLTISSLQAEDVAVYYCQNVYSYPLTFGQGTKLEIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 88
EVQLVESGGGLVQPGGSLRLSCAASGFTLNNFGMHWVRQAPGKGLEWVSYISSGSGTIYYADSV
KGRFTISRDNPKNSLYLQMNSLRAEDTAVYYCARAYYGNGFSFWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 89
DIVMTQSPDSLAVSLGERATINCKSSQSLLNSGNQKNYLTWYQQKPGQPPKLLIYWASTRESGV
PDRFSGSGSGTDFTLTISSLQAEDVAVYYCQNVYSYPLTFGQGTKLEIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 90
QIQLVQSGSELKKPGASVKVSCKASGYTFTNYGMNWVRQAPGQGLEWMGWINTYTGESTYADGF
TGRFVFSSDTSARTAYLQISSLKAEDTAVYFCATLGYGNSLAYWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 91

DIVMTQSPDSLAVTTGERATINCKSSQSLLNSGNQRNYLTWYQQKPGQPPKLLIYWASTRESGV
PDRFSGSGSGTDFTLTISSLQAEDVAVYYCQNNYFYPFTFGQGTKLEIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 92
QVQLVQSGSELKKPGESVKVSCKASGYTFTNYGMNWVRQAPGQGLEWMGWINTYTGESTYADGF
TGRFVFSMDTSASTAYLQISSLKAEDMAMYFCATLGYGNSLAYWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 93
DIVMTQSPDSLAVSLGERATINCKSSQSLLNSGNQRNYLTWYQQKPGQPPKLLIYWASTRESGV
PDRFSGSGSGTDFTLTISSLQAEDVAVYYCQNNYFYPFTFGQGTKLEIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 94
QIQLVQSGSELKKPGESVKVSCKASGYTFTNYGMNWVRQAPGQGLEWMGWINTYTGESTYADGF
TGRFVFSSDTSARTAYLQISSLKAEDTAVYFCATLGYGNSLAYWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 95
DIVMTQSPDFLAVSLGERATINCKSSQSLLNSGNQKNYLTWYQQKPGQPPKLLIYWASTRDSGV
PDRFSGSGSGTDFTLTISSLQAEDVAVYYCQNAYFYPFTFGPGTKVDIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 96
QVQLVQSGSELKKPGESVKVSCKASGYTFTNYGMNWVRQAPGQGLEWMGWINTYTGESTYADGF
TGRFVFSMDTSASTAYLQISSLKAEDMAMYFCATLGYGNSLAYWGQGTLVTVSSASTKGPSVFP
LAPSSKSTSGGTAALGCLVKDYFPEPVTVSWNSGALTSGVHTFPAVLQSSGLYSLSSVVTVPSS

SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 97
DIVMTQSPDSLAVSLGERATINCKSSQSLLNSGNQRNYLTWYQQKPGQPPKLLIYWASTRESGV
PDRFSGSGSGTDFTLTISSLQAEDVAVYYCQNNYFYPFTFGQGTKLEIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 98
EVQLVESGGGLVKPGGSLRLSCAASGFTFSSYGMSWVRQAPGKGLEWVSTISGGDSYTYYPDSV
KGRFTISRDIAKNSLYLQMNSLRAEDTAVYYCARSYNANSLPYWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 99
DIVMTQSPDSLAVSLGERATINCKSSQSLLNSGNQRNYLTWYQQKPGQPPKLLIYWASTRESGV
PDRFSGSGSGTDFTLTISSLQAEDVAVYYCQNNYFYPFTFGQGTKLEIKRTVAAPSVFIFPPSD
EQLKSGTASVVCLLNNFYPREAKVQWKVDNALQSGNSQESVTEQDSKDSTYSLSSTLTLSKADY
EKHKVYACEVTHQGLSSPVTKSFNRGEC

SEQ ID NO: 100
EVQLVESGGGLVKPGGSLRLSCAASGFTFSSYGMSWVRQAPGKGLEWVSTISGGDSYTYYPDSV
KGRFTISRDNAKNSLYLQMNSLRAEDTAVYYCARSYNGNSLPYWGQGTLVTVSSASTKGPSVFP
LAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHNHYTQKSLSLSPGK

SEQ ID NO: 101
DIVMTQSPDSLAVSLGERATINCKSSQSLLNSGNQRNYLTWYQQKPGQPPKLLIYWASTRESGV
PDRFSGSGSGTDFTLTISSLQAEDVAVYYCQNNYFYPFTFGQGTKLEIKRTVAAPSVFIFPPSD

EQLKSGTASVVCLLNNFYPREAKVQWKVDNALQSGNSQESVTEQDSKDSTYSLSSTLTLSKADY
EKHKVYACEVTHQGLSSPVTKSFNRGEC

## Claims

1. An anti-CLDN-18.2 antibody or an antigen binding fragment thereof comprising a heavy chain variable region and a light chain variable region, **characterized in that**,
the heavy chain variable region comprises HCDR1, HCDR2 and HCDR3, wherein
the sequence of the HCDR1 is selected from amino acid sequences as shown in SEQ ID NOs: 1, 10, 16, 22, 28, 34 and 37;
the sequence of the HCDR2 is selected from amino acid sequences as shown in SEQ ID NOs: 62, 63, 17, 23, 29, 35, 38, 72 and 74; in the HCDR2 as shown in SEQ ID NO: 62, X₁ is C or S, and X₂ is T or S, and in the HCDR2 as shown in SEQ ID NO: 63, X₃ is D or G, and X₄ is K or T; and
the sequence of the HCDR3 is selected from amino acid sequences as shown in SEQ ID NOs: 3, 12, 18, 24, 30, 36, 39, 73 and 75; and
the light chain variable region comprises LCDR1, LCDR2 and LCDR3, wherein
the sequence of the LCDR1 is selected from amino acid sequences as shown in SEQ ID NOs: 4, 7, 13, 19, 25 and 31;
the sequence of the LCDR2 is selected from amino acid sequences as shown in SEQ ID NOs: 5, 8, 14, 20, 26 and 32; and
the sequence of the LCDR3 is selected from amino acid sequences as shown in SEQ ID NOs: 6, 9, 15, 21, 27 and 33.

2. The antibody or the antigen binding fragment thereof of claim 1, **characterized in that**
the heavy chain variable region comprises:
(I) HCDR1, HCDR2 and HCDR3 with amino acid sequences as shown in SEQ ID NO: 1, SEQ ID NO: 2 and SEQ ID NO: 3, respectively; or
(II) HCDR1, HCDR2 and HCDR3 with amino acid sequences as shown in SEQ ID NO: 10, SEQ ID NO: 11 and SEQ ID NO: 12, respectively; or
(III) HCDR1, HCDR2 and HCDR3 with amino acid sequences as shown in SEQ ID NO: 16, SEQ ID NO: 17 and SEQ ID NO: 18, respectively; or
(IV) HCDR1, HCDR2 and HCDR3 with amino acid sequences as shown in SEQ ID NO: 22, SEQ ID NO: 23 and SEQ ID NO: 24, respectively; or
(V) HCDR1, HCDR2 and HCDR3 with amino acid sequences as shown in SEQ ID NO: 28, SEQ ID NO: 29 and SEQ ID NO: 30, respectively; or
(VI) HCDR1, HCDR2 and HCDR3 with amino acid sequences as shown in SEQ ID NO: 34, SEQ ID NO: 35 and SEQ ID NO: 36, respectively; or
(VII) HCDR1, HCDR2 and HCDR3 with amino acid sequences as shown in SEQ ID NO: 37, SEQ ID NO: 38 and SEQ ID NO: 39, respectively; or
(VIII) HCDR1, HCDR2 and HCDR3 with amino acid sequences as shown in SEQ ID NO: 10, SEQ ID NO: 40 and SEQ ID NO: 12, respectively; or
(IX) HCDR1, HCDR2 and HCDR3 with amino acid sequences as shown in SEQ ID NO: 1, SEQ ID NO: 41 and SEQ ID NO: 3, respectively; or
(X) HCDR1, HCDR2 and HCDR3 with amino acid sequences as shown in SEQ ID NO: 1, SEQ ID NO: 72 and SEQ ID NO: 73, respectively; or
(XI) HCDR1, HCDR2 and HCDR3 with amino acid sequences as shown in SEQ ID NO: 1, SEQ ID NO: 72 and SEQ ID NO: 3, respectively; or
(XII) HCDR1, HCDR2 and HCDR3 with amino acid sequences as shown in SEQ ID NO: 28, SEQ ID NO: 74 and SEQ ID NO: 75, respectively; or
(XIII) HCDR1, HCDR2 and HCDR3 with amino acid sequences as shown in SEQ ID NO: 28, SEQ ID NO: 74 and SEQ ID NO: 30, respectively; and
the light chain variable region comprises:
(I) LCDR1, LCDR2 and LCDR3 with amino acid sequences as shown in SEQ ID NO: 4, SEQ ID NO: 5 and SEQ ID NO: 6, respectively; or
(II) LCDR1, LCDR2 and LCDR3 with amino acid sequences as shown in SEQ ID NO: 7, SEQ ID NO: 8 and SEQ ID NO: 9, respectively; or
(III) LCDR1, LCDR2 and LCDR3 with amino acid sequences as shown in SEQ ID NO: 13, SEQ ID NO: 14 and SEQ ID NO: 15, respectively; or
(IV) LCDR1, LCDR2 and LCDR3 with amino acid sequences as shown in SEQ ID NO: 19, SEQ ID NO: 20 and SEQ ID NO: 21, respectively; or
(V) LCDR1, LCDR2 and LCDR3 with amino acid sequences as shown in SEQ ID NO: 25, SEQ ID NO: 26 and SEQ ID NO: 27, respectively; or
(VI) LCDR1, LCDR2 and LCDR3 with amino acid sequences as shown in SEQ ID NO: 31, SEQ ID NO: 32 and SEQ ID NO: 33, respectively.

3. The antibody or the antigen binding fragment thereof of claim 2, **characterized in** comprising:
(I) a heavy chain variable region comprising HCDR1, HCDR2 and HCDR3 with amino acid sequences as shown in SEQ ID NO: 1, SEQ ID NO: 2 and SEQ ID NO: 3, respectively; and a light chain variable region comprising LCDR1, LCDR2 and LCDR3 with amino acid sequences as shown in SEQ ID NO: 4, SEQ ID NO: 5 and SEQ ID NO: 6, respectively; or
(II) a heavy chain variable region comprising HCDR1, HCDR2 and HCDR3 with amino acid sequences as shown in SEQ ID NO: 1, SEQ ID NO: 2 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(III) a heavy chain variable region comprising HCDR1, HCDR2 and HCDR3 with amino acid sequences as shown in SEQ ID NO: 10, SEQ ID NO: 11 and SEQ ID NO: 12, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(IV) a heavy chain variable region comprising HCDR1, HCDR2 and HCDR3 with amino acid sequences as shown in SEQ ID NO: 16, SEQ ID NO: 17 and SEQ ID NO: 18, respectively; and a light chain variable region comprising LCDR1, LCDR2 and LCDR3 with amino acid sequences as shown in SEQ ID NO: 19, SEQ ID NO: 20 and SEQ ID NO: 21, respectively; or
(V) a heavy chain variable region comprising HCDR1, HCDR2 and HCDR3 with amino acid sequences as shown in SEQ ID NO: 22, SEQ ID NO: 23 and SEQ ID NO: 24, respectively; and a light chain variable region comprising LCDR1, LCDR2 and LCDR3 with amino acid sequences as shown in SEQ ID NO: 25, SEQ ID NO: 26 and SEQ ID NO: 27, respectively; or
(VI) a heavy chain variable region comprising HCDR1, HCDR2 and HCDR3 with amino acid sequences as shown in SEQ ID NO: 28, SEQ ID NO: 29 and SEQ ID NO: 30, respectively; and a light chain variable region comprising LCDR1, LCDR2 and LCDR3 with amino acid sequences as shown in SEQ ID NO: 31, SEQ ID NO: 32 and SEQ ID NO: 33, respectively; or
(VII) a heavy chain variable region comprising HCDR1, HCDR2 and HCDR3 with amino acid sequences as shown in SEQ ID NO: 34, SEQ ID NO: 35 and SEQ ID NO: 36, respectively; and a light chain variable region comprising LCDR1, LCDR2 and LCDR3 with amino acid sequences as shown in SEQ ID NO: 25, SEQ ID NO: 26 and SEQ ID NO: 27, respectively; or
(VIII) a heavy chain variable region comprising HCDR1, HCDR2 and HCDR3 with amino acid sequences as shown in SEQ ID NO: 37, SEQ ID NO: 38 and SEQ ID NO: 39, respectively; and a light chain variable region comprising LCDR1, LCDR2 and LCDR3 with amino acid sequences as shown in SEQ ID NO: 31, SEQ ID NO: 32 and SEQ ID NO: 33, respectively; or
(IX) a heavy chain variable region comprising HCDR1, HCDR2 and HCDR3 with amino acid sequences as shown in SEQ ID NO: 10, SEQ ID NO: 11 and SEQ ID NO: 12, respectively; and a light chain variable region comprising LCDR1, LCDR2 and LCDR3 with amino acid sequences as shown in SEQ ID NO: 19, SEQ ID NO: 20 and SEQ ID NO: 21, respectively; or
(X) a heavy chain variable region comprising HCDR1, HCDR2 and HCDR3 with amino acid sequences as shown in SEQ ID NO: 28, SEQ ID NO: 29 and SEQ ID NO: 30, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XI) a heavy chain variable region comprising HCDR1, HCDR2 and HCDR3 with amino acid sequences as shown in SEQ ID NO: 22, SEQ ID NO: 23 and SEQ ID NO: 24, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XII) a heavy chain variable region comprising HCDR1, HCDR2 and HCDR3 with amino acid sequences as shown in SEQ ID NO: 10, SEQ ID NO: 40 and SEQ ID NO: 12, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XIII) a heavy chain variable region comprising HCDR1, HCDR2 and HCDR3 with amino acid sequences as shown in SEQ ID NO: 1, SEQ ID NO: 41 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(XIV) a heavy chain variable region comprising HCDR1, HCDR2 and HCDR3 with amino acid sequences as shown in SEQ ID NO: 1, SEQ ID NO: 72 and SEQ ID NO: 73, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(XV) a heavy chain variable region comprising HCDR1, HCDR2 and HCDR3 with amino acid sequences as shown in SEQ ID NO: 1, SEQ ID NO: 72 and SEQ ID NO: 3, respectively; and a light chain variable region comprising LCDR1, LCDR2 and LCDR3 with amino acid sequences as shown in SEQ ID NO: 7, SEQ ID NO: 8 and SEQ ID NO: 9, respectively; or
(XVI) a heavy chain variable region comprising HCDR1, HCDR2 and HCDR3 with amino acid sequences as shown in SEQ ID NO: 28, SEQ ID NO: 74 and SEQ ID NO: 30, respectively; and a light chain variable region comprising LCDR1, LCDR2 and LCDR3 with amino acid sequences as shown in SEQ ID NO: 13, SEQ ID NO: 14 and SEQ ID NO: 15, respectively; or
(XVII) a heavy chain variable region comprising HCDR1, HCDR2 and HCDR3 with amino acid sequences as shown in SEQ ID NO: 28, SEQ ID NO: 74 and SEQ ID NO: 75, respectively; and a light chain variable region comprising LCDR1, LCDR2 and LCDR3 with amino acid sequences as shown in SEQ ID NO: 13, SEQ ID NO: 14 and SEQ ID NO: 15, respectively.

4. The antibody or the antigen binding fragment thereof of any one of claims 1-3, comprising a heavy chain variable region and a light chain variable region, **characterized in that**
(1) the amino acid sequence of the heavy chain variable region comprises a sequence selected from SEQ ID NOs: 42, 45, 47, 49, 51, 53, 54, 76, 78, 82 and 83, or an amino acid sequence having at least 95%, 96%, 97%, 98%, or 99% identity to any of the sequences as shown in SEQ ID NOs: 42, 45, 47, 49, 51, 53, 54, 76, 78, 82 and 83; and
the amino acid sequence of the light chain variable region comprises a sequence selected from SEQ ID NOs: 43, 44, 46, 48, 50 and 52, or an amino acid sequence having at least 95%, 96%, 97%, 98%, or 99% identity to any of the sequences as shown in SEQ ID NOs: 43, 44, 46, 48, 50 and 52; or
(2) the antibody or the antigen binding fragment thereof comprises:
a heavy chain variable region with an amino acid sequence as shown in SEQ ID NO: 42 and a light chain variable region with an amino acid sequence as shown in SEQ ID NO: 43; or
a heavy chain variable region with an amino acid sequence as shown in SEQ ID NO: 42 and a light chain variable region with an amino acid sequence as shown in SEQ ID NO: 44; or
a heavy chain variable region with an amino acid sequence as shown in SEQ ID NO: 45 and a light chain variable region with an amino acid sequence as shown in SEQ ID NO: 46; or
a heavy chain variable region with an amino acid sequence as shown in SEQ ID NO: 47 and a light chain variable region with an amino acid sequence as shown in SEQ ID NO: 48; or
a heavy chain variable region with an amino acid sequence as shown in SEQ ID NO: 49 and a light chain variable region with an amino acid sequence as shown in SEQ ID NO: 50; or
a heavy chain variable region with an amino acid sequence as shown in SEQ ID NO: 51 and a light chain variable region with an amino acid sequence as shown in SEQ ID NO: 52; or
a heavy chain variable region with an amino acid sequence as shown in SEQ ID NO: 53 and a light chain variable region with an amino acid sequence as shown in SEQ ID NO: 50; or
a heavy chain variable region with an amino acid sequence as shown in SEQ ID NO: 54 and a light chain variable region with an amino acid sequence as shown in SEQ ID NO: 52; or
a heavy chain variable region with an amino acid sequence as shown in SEQ ID NO: 45 and a light chain variable region with an amino acid sequence as shown in SEQ ID NO: 48; or
a heavy chain variable region with an amino acid sequence as shown in SEQ ID NO: 51 and a light chain variable region with an amino acid sequence as shown in SEQ ID NO: 46; or
a heavy chain variable region with an amino acid sequence as shown in SEQ ID NO: 49 and a light chain variable region with an amino acid sequence as shown in SEQ ID NO: 46.

5. The antibody or the antigen binding fragment thereof of any one of claims 1-3, comprising a heavy chain variable region and a light chain variable region, **characterized in that**
(1) the amino acid sequence of the heavy chain variable region comprises a sequence selected from SEQ ID NOs: 55, 57, 58, 59, 60, 76, 78, 79, 82 and 83, or an amino acid sequence having at least 95%, 96%, 97%, 98%, or 99% identity to any of the sequences as shown in SEQ ID NOs: 55, 57, 58, 59, 60, 76, 78, 79, 82 and 83; and
the amino acid sequence of the light chain variable region comprises a sequence selected from SEQ ID NOs: 56, 61, 77, 80 and 81, or an amino acid sequence having at least 95%, 96%, 97%, 98%, or 99% identity to any of the sequences as shown in SEQ ID NOs: 56, 61, 77, 80 and 81; or
(2) the heavy chain variable region comprises an amino acid sequence as shown in any one of SEQ ID NO: 55, 57, 60, 76, 78, 79, 82 or 83; and the light chain variable region comprises an amino acid sequence as shown in any one of SEQ ID NO: 56, 61, 77, 80 or 81; or
(3) the heavy chain variable region comprises an amino acid sequence as shown in SEQ ID NO: 55, 57, 82 or 83; and the light chain variable region comprises an amino acid sequence as shown in SEQ ID NO: 56; or
(4) the heavy chain variable region comprises an amino acid sequence as shown in SEQ ID NO: 60, 76 or 78; and the light chain variable region comprises an amino acid sequence as shown in SEQ ID NO: 61; or
(5) the heavy chain variable region comprises an amino acid sequence as shown in SEQ ID NO: 55; and the light chain variable region comprises an amino acid sequence as shown in SEQ ID NO: 56; or
(6) the heavy chain variable region comprises an amino acid sequence as shown in SEQ ID NO: 57; and the light chain variable region comprises an amino acid sequence as shown in SEQ ID NO: 56; or
(7) the heavy chain variable region comprises an amino acid sequence as shown in SEQ ID NO: 58; and the light chain variable region comprises an amino acid sequence as shown in SEQ ID NO: 56; or
(8) the heavy chain variable region comprises an amino acid sequence as shown in SEQ ID NO: 59; and the light chain variable region comprises an amino acid sequence as shown in SEQ ID NO: 56; or
(9) the heavy chain variable region comprises an amino acid sequence as shown in SEQ ID NO: 60; and the light chain variable region comprises an amino acid sequence as shown in SEQ ID NO: 61; or
(10) the heavy chain variable region comprises an amino acid sequence as shown in SEQ ID NO: 76; and the light chain variable region comprises an amino acid sequence as shown in SEQ ID NO: 77; or
(11) the heavy chain variable region comprises an amino acid sequence as shown in SEQ ID NO: 79; and the light chain variable region comprises an amino acid sequence as shown in SEQ ID NO: 80; or
(12) the heavy chain variable region comprises an amino acid sequence as shown in SEQ ID NO: 55; and the light chain variable region comprises an amino acid sequence as shown in SEQ ID NO: 81.

6. The antibody or the antigen binding fragment thereof of any one of claims 1-3, comprising a heavy chain and a light chain, **characterized in that**
(1) the amino acid sequence of the heavy chain is as shown in SEQ ID NO: 64 or a variant thereof or SEQ ID NO: 68 or a variant thereof, and the amino acid sequence of the light chain is as shown in SEQ ID NO: 65 or a variant thereof or SEQ ID NO: 69 or a variant thereof, wherein the variant comprises 1, 2, 3, 4 or 5 amino acid changes in its variable region; preferably, the variant of SEQ ID NO: 64 comprises an amino acid change at position 50 or 63 or a combination thereof; and/or the variant of SEQ ID NO: 68 comprises an amino acid change at position 63 or 65 or a combination thereof; preferably, the variant of SEQ ID NO: 64 comprises S50C or S63T or a combination thereof; and/or the variant of SEQ ID NO: 68 comprises G63D or T65K or a combination thereof; or
(2) the heavy chain comprises an amino acid sequence as shown in SEQ ID NO: 64, or an amino acid sequence having at least 90%, 92%, 94%, 95%, 96%, 97%, 98% or 99% sequence identity to the amino acid sequence as shown in SEQ ID NO: 64; and the light chain comprises an amino acid sequence as shown in SEQ ID NO: 65, or an amino acid sequence having at least 90%, 92%, 94%, 95%, 96%, 97%, 98% or 99% sequence identity to the amino acid sequence as shown in SEQ ID NO: 65; or
(3) the heavy chain comprises an amino acid sequence as shown in SEQ ID NO: 68, or an amino acid sequence having at least 90%, 92%, 94%, 95%, 96%, 97%, 98% or 99% sequence identity to the amino acid sequence as shown in SEQ ID NO: 68; and the light chain comprises an amino acid sequence as shown in SEQ ID NO: 69, or an amino acid sequence having at least 90%, 92%, 94%, 95%, 96%, 97%, 98% or 99% sequence identity to the amino acid sequence as shown in SEQ ID NO: 69; or
(4) the heavy chain comprises an amino acid sequence as shown in SEQ ID NO: 84, or an amino acid sequence having at least 90%, 92%, 94%, 95%, 96%, 97%, 98% or 99% sequence identity to the amino acid sequence as shown in SEQ ID NO: 84; and the light chain comprises an amino acid sequence as shown in SEQ ID NO: 85, or an amino acid sequence having at least 90%, 92%, 94%, 95%, 96%, 97%, 98% or 99% sequence identity to the amino acid sequence as shown in SEQ ID NO: 85; or
(5) the heavy chain comprises an amino acid sequence as shown in SEQ ID NO: 86, or an amino acid sequence having at least 90%, 92%, 94%, 95%, 96%, 97%, 98% or 99% sequence identity to the amino acid sequence as shown in SEQ ID NO: 86; and the light chain comprises an amino acid sequence as shown in SEQ ID NO: 87, or an amino acid sequence having at least 90%, 92%, 94%, 95%, 96%, 97%, 98% or 99% sequence identity to the amino acid sequence as shown in SEQ ID NO: 87; or
(6) the heavy chain comprises an amino acid sequence as shown in SEQ ID NO: 88, or an amino acid sequence having at least 90%, 92%, 94%, 95%, 96%, 97%, 98% or 99% sequence identity to the amino acid sequence as shown in SEQ ID NO: 88; and the light chain comprises an amino acid sequence as shown in SEQ ID NO: 89, or an amino acid sequence having at least 90%, 92%, 94%, 95%, 96%, 97%, 98% or 99% sequence identity to the amino acid sequence as shown in SEQ ID NO: 89; or
(7) the heavy chain comprises an amino acid sequence as shown in SEQ ID NO: 90, or an amino acid sequence having at least 90%, 92%, 94%, 95%, 96%, 97%, 98% or 99% sequence identity to the amino acid sequence as shown in SEQ ID NO: 90; and the light chain comprises an amino acid sequence as shown in SEQ ID NO: 91, or an amino acid sequence having at least 90%, 92%, 94%, 95%, 96%, 97%, 98% or 99% sequence identity to the amino acid sequence as shown in SEQ ID NO: 91; or
(8) the heavy chain comprises an amino acid sequence as shown in SEQ ID NO: 92, or an amino acid sequence having at least 90%, 92%, 94%, 95%, 96%, 97%, 98% or 99% sequence identity to the amino acid sequence as shown in SEQ ID NO: 92; and the light chain comprises an amino acid sequence as shown in SEQ ID NO: 93, or an amino acid sequence having at least 90%, 92%, 94%, 95%, 96%, 97%, 98% or 99% sequence identity to the amino acid sequence as shown in SEQ ID NO: 93; or
(9) the heavy chain comprises an amino acid sequence as shown in SEQ ID NO: 94, or an amino acid sequence having at least 90%, 92%, 94%, 95%, 96%, 97%, 98% or 99% sequence identity to the amino acid sequence as shown in SEQ ID NO: 94; and the light chain comprises an amino acid sequence as shown in SEQ ID NO: 95, or an amino acid sequence having at least 90%, 92%, 94%, 95%, 96%, 97%, 98% or 99% sequence identity to the amino acid sequence as shown in SEQ ID NO: 95; or
(10) the heavy chain comprises an amino acid sequence as shown in SEQ ID NO: 96, or an amino acid sequence having at least 90%, 92%, 94%, 95%, 96%, 97%, 98% or 99% sequence identity to the amino acid sequence as shown in SEQ ID NO: 96; and the light chain comprises an amino acid sequence as shown in SEQ ID NO: 97, or an amino acid sequence having at least 90%, 92%, 94%, 95%, 96%, 97%, 98% or 99% sequence identity to the amino acid sequence as shown in SEQ ID NO: 97; or
(11) the heavy chain comprises an amino acid sequence as shown in SEQ ID NO: 98, or an amino acid sequence having at least 90%, 92%, 94%, 95%, 96%, 97%, 98% or 99% sequence identity to the amino acid sequence as shown in SEQ ID NO: 98; and the light chain comprises an amino acid sequence as shown in SEQ ID NO: 99, or an amino acid sequence having at least 90%, 92%, 94%, 95%, 96%, 97%, 98% or 99% sequence identity to the amino acid sequence as shown in SEQ ID NO: 99; or
(12) the heavy chain comprises an amino acid sequence as shown in SEQ ID NO: 100, or an amino acid sequence having at least 90%, 92%, 94%, 95%, 96%, 97%, 98% or 99% sequence identity to the amino acid sequence as shown in SEQ ID NO: 100; and the light chain comprises an amino acid sequence as shown in SEQ ID NO: 101, or an amino acid sequence having at least 90%, 92%, 94%, 95%, 96%, 97%, 98% or 99% sequence identity to the amino acid sequence as shown in SEQ ID NO: 101.

7. The antibody or the antigen binding fragment thereof of any one of claims 1-6, **characterized in that** the antibody is a murine antibody, a chimeric antibody, a humanized antibody, or a fully human antibody; the antigen binding fragment is an Fab, an Fab', an Fab'-SH, an Fv, an scFv, an F(ab')2, an sdAb or a diabody.

8. The antibody or the antigen binding fragment thereof of any one of claims 1-6, **characterized in that** the antibody is of any IgG subtype, such as IgG1, IgG2, IgG3 or IgG4; preferably, the antibody is hypofucosylated or afucosylated.

9. An isolated anti-CLDN-18.2 antibody or an antigen binding fragment thereof having one or more of the following properties:
(1) binding to the same or completely or partially overlapping epitope of human CLDN-18.2 protein as the anti-CLDN-18.2 antibody or the antigen binding fragment thereof of any one of claims 1-8;
(2) competing with the anti-CLDN-18.2 antibody or the antigen binding fragment thereof of any one of claims 1-8 to bind to an epitope of human CLDN-18.2 protein;
(3) binding to human CLDN-18.2 protein, but not to human CLDN-18.1 protein;
(4) inducing the ADCC effect of cells expressing human CLDN-18.2 protein; and
(5) inducing the CDC effect of cells expressing human CLDN-18.2 protein.

10. A polynucleotide coding the anti-CLDN-18.2 antibody or the antigen binding fragment thereof of any one of claims 1-9.

11. An expression vector comprising the polynucleotide of claim 10, **characterized in that** the vector is preferably a eukaryotic expression vector.

12. A host cell comprising the polynucleotide of claim 10 or the expression vector of claim 11, or expressing the anti-CLDN-18.2 antibody or the antigen binding fragment thereof of any one of claims 1-9, **characterized in that** the host cell is preferably a eukaryotic cell, and more preferably a mammalian cell.

13. A method for preparing the anti-CLDN-18.2 antibody or the antigen binding fragment thereof of any one of claims 1-6, **characterized in** comprising culturing the host cell of claim 12 under conditions suitable for expression of the antibody or the antigen binding fragment thereof, and recovering the expressed antibody or the antigen binding fragment thereof from the host cell.

14. A pharmaceutical composition comprising the anti-CLDN-18.2 antibody or the antigen binding fragment thereof of any one of claims 1-9, the polynucleotide of claim 10, the expression vector of claim 11, the host cell of claim 12, and a pharmaceutically acceptable carrier or excipient.

15. Use of the antibody or the antigen binding fragment thereof of any one of claims 1-9, the polynucleotide of claim 10, the expression vector of claim 11, the host cell of claim 12, or the pharmaceutical composition of claim 14 in the preparation of a medicament for the treatment and/or prevention of a disease or condition mediated by CLDN-18.2, **characterized in that** the disease or condition is preferably a cancer;
and more preferably, the cancer is selected from gastric cancer, esophageal cancer, gastroesophageal cancer, pancreatic cancer, cholangiocarcinoma, lung cancer, ovarian cancer, colon cancer, liver cancer, head and neck cancer, gallbladder cancer, intestinal cancer, and bladder cancer.

16. A pharmaceutical combination comprising the antibody or the antigen binding fragment thereof of any one of claims 1-9, the polynucleotide of claim 10, the expression vector of claim 11, the host cell of claim 12, or the pharmaceutical composition of claim 14, and one or more additional therapeutic agents.
